# EUROPÄISCHE PATENTSCHRIFT

(11) **EP 0 818 447 B1**
(45) Veröffentlichungstag und Bekanntmachung des Hinweises auf die Patenterteilung: **26.05.2004**
(21) Anmeldenummer: 97110276.9
(22) Anmeldetag: 24.06.1997
(51) Int. Cl.: C07D 213/30, A61K 31/44

(54) **6-(Hydroxymethyl-ethyl)pyridine als HMG-CoA-Reduktase Inhibitoren**
6-(Hydroxymethyl-ethyl)pyridines as HMG-CoA-reductase inhibitors
6-(Hydroxyméthyl-éthyl)pyridines comme inhibiteurs de HMG-CoA réductase

(30) Priorität: 08.07.1996 DE 19627420
(43) Veröffentlichungstag der Anmeldung: 14.01.1998
(73) Patentinhaber: Bayer HealthCare AG, 51368 Leverkusen (DE)
(72) Erfinder: Fey, Peter, Dr., 42111 Wuppertal (DE); Angerbauer, Rolf, Dr., Higashinada-ku, Kobe-shi (JP); Schmidt, Delf, Dr., 42113 Wuppertal (DE); Bischoff, Hilmar, Dr., 42113 Wuppertal (DE); Kanhai, Wolfgang, Dr., 42327 Wuppertal (DE); Radtke, Martin, Dr., 40699 Erkrath (DE); Karl, Wolfgang, Dr., 51519 Odenthal (DE)

(56) Entgegenhaltungen:
- EP-A- 0 325 130
- EP-A- 0 491 226
- EP-A- 0 603 699
- DE-A- 4 321 421

## Beschreibung

Die vorliegende Erfindung betrifft 6-(Hydroxymethyl-ethyl)pyridine, ein Verfahren zu ihrer Herstellung und ihre Verwendung als Arzneimitteln, insbesondere als antiatherosklerotische Mittel.

Es ist bekannt, daß aus Pilzkulturen isolierte Lacton-Derivate Inhibitoren der 3-Hydroxy-3-methyl-glutaryl-CoenzymA Reduktase (HMG-CoA-Reduktase) sind [Mevinolin, EP22478; US-4231938].

Außerdem ist bekannt, daß Pyridin-substituierte Dihydroxyheptensäuren Inhibitoren der HMG-CoA-Reduktase sind [EP325130; EP307342; EP306929].

Es wurde nun gefunden, daß die 6-(Hydroxymethyl-ethyl)pyridine der allgemeinen Formel (I) 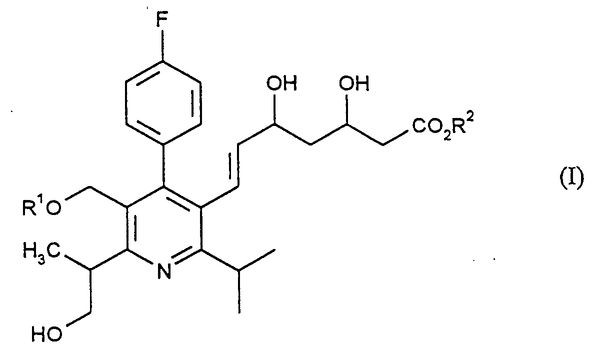 in welcher
- R¹: für Wasserstoff oder für Methyl steht,
und
- R²: für Wasserstoff oder für Methyl steht,
und deren Salze, gegebenenfalls in einer isomeren Form, eine überlegene inhibitorische Wirkung auf die HMG-CoA-Reduktase aufweisen und somit eine überraschend gute Senkung des Cholesteringehalts im Blut bewirken.

Die erfindungsgemäßen Verbindungen können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze der erfindungsgemäßen 6-(Hydroxymethyl-ethyl)pyridine können Metall- oder Ammoniumsalze sein. Bevorzugt zu nennen seien Natrium-, Kalium-, Magnesium- oder Calciumsalze sowie Ammoniumsalze, die abgeleitet sind von Ammoniak oder organischen Aminen wie beispielsweise Methylamin, Ethylamin, Propylamin, Isopropylamin, Di- bzw. Triethylamin, Diisopropylamin, Di- bzw. Triethanolamin, Dicyclohexylamin, Arginin, Lysin oder Ethylendiamin. Besonders bevorzugt sind Natrium- und Kaliumsalze.

Die erfindungsgemäßen Verbindungen sowie deren Salze haben 3 asymmetrische Kohlenstoffatome, nämlich die beiden Kohlenstoffatome der Seitenkette, an denen die Hydroxygruppen und das Kohlenstoffatom, an dem die Hydroxymethylgruppe gebunden sind.

Sie können daher in verschiedenen stereochemischen Formen existieren. Die Erfindung betrifft sowohl die einzelnen Isomeren als auch deren Mischungen. So können die erfindungsgemäßen Verbindungen je nach der relativen Stellung der Hydroxygruppen in der Erythro-Konfiguration oder in der Threo-Konfiguration vorliegen.

Folgendes Formelschema erläutert dies beispielhaft:

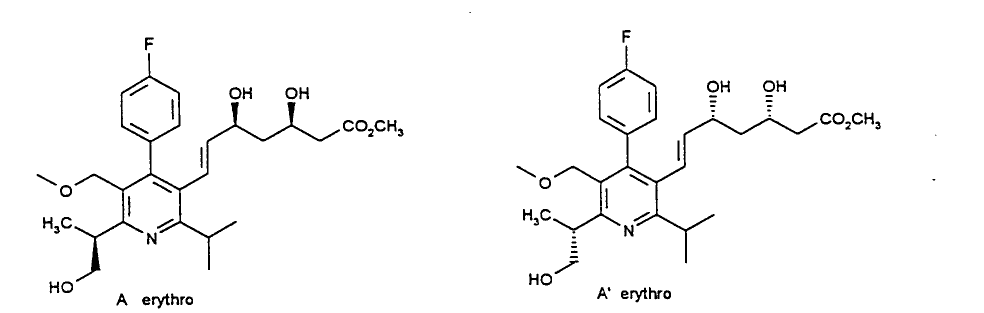

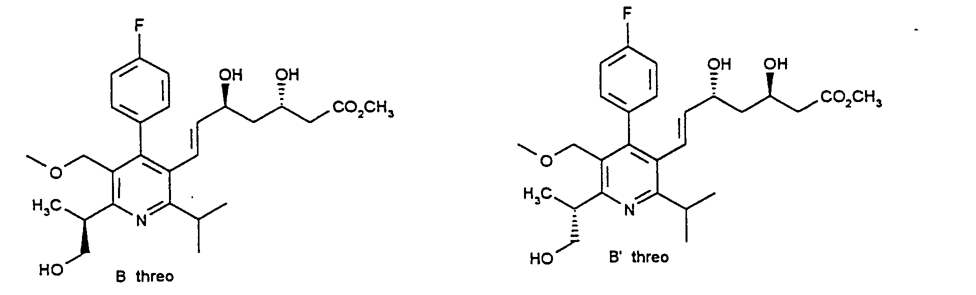

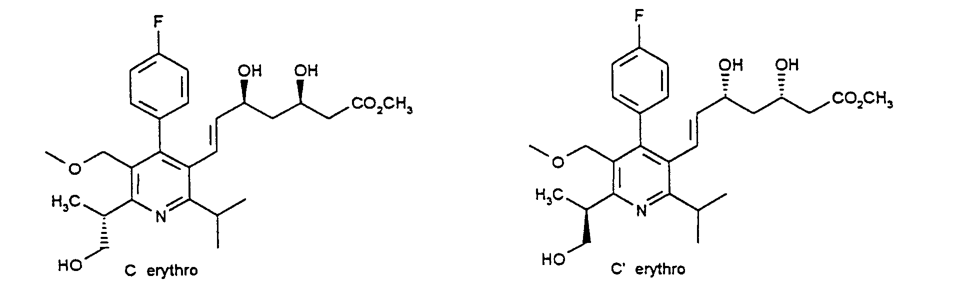

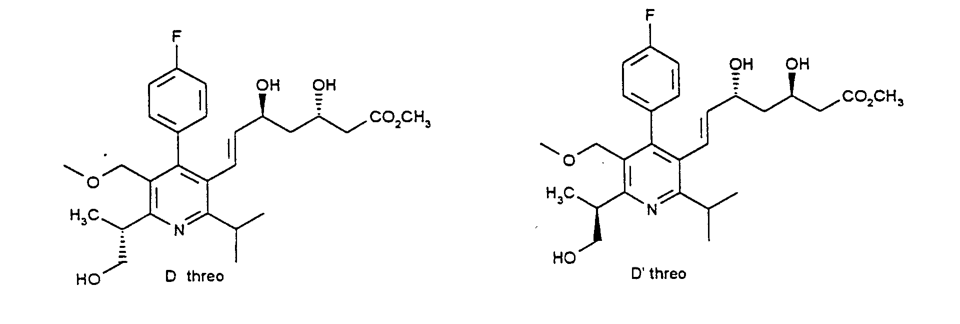

Bevorzugt ist die Erythro-Konfiguration.

Sowohl von den Stoffen in Threo- als auch in Erythro-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form). Bevorzugt sind hiervon die 3R,5S/3S,5R-Racemate sowie die 3R,5S-Enantiomere.

Darüber hinaus können die erfindungsgemäßen Stoffe aufgrund der Doppelbindung in E-Konfiguration oder der Z-Konfiguration vorliegen. Bevorzugt sind diejenigen Verbindungen, die E-konfiguriert sind.

Besonders bevorzugt sind die 1S und 1R-Enantiomere der (3R,5S)-Dihydroxyheptensäuren und Derivate in der Erythro-(E)-Konfiguration sowie deren Salze.

Ganz besonders bevorzugt sind die erythro-konfigurierte Natriumsalze der erfindungsgemäßen Verbindungen.

Die erfindungsgemäßen Verbindungen der allgemeinen Formel (I) können hergestellt werden, indem man

Verbindungen der allgemeinen Formel (II) 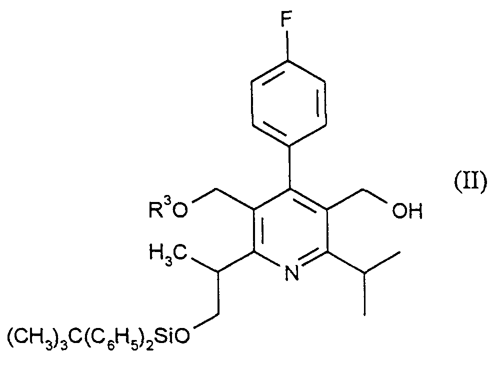 in welcher
- R³: für Methyl oder für den Rest -Si(CH₃)₂C(CH₃)₃ (TBDMS) steht,
zunächst mit Aluminiumoxid und Pyridiniumchlorochromat in inerten Lösemitteln zu den Aldehyden der allgemeinen Formel (III) 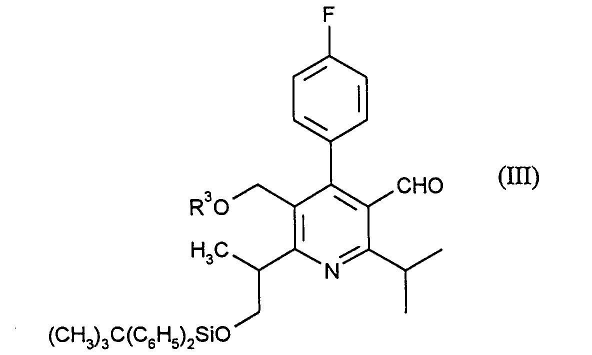 in welcher
- R³: die oben angegebene Bedeutung hat,
oxidiert,
aus diesen in einem zweiten Schritt durch Umsetzung mit dem Ketophosphonat (CH₃O)₂PO-CH₂-CO-CH₂-CH(OSi(CH₃)₂)C(CH₃)₃)-CH₂-CO₂CH₃ in Anwesenheit von Basen und Lösemitteln
die Verbindungen der allgemeinen Formel (IV) 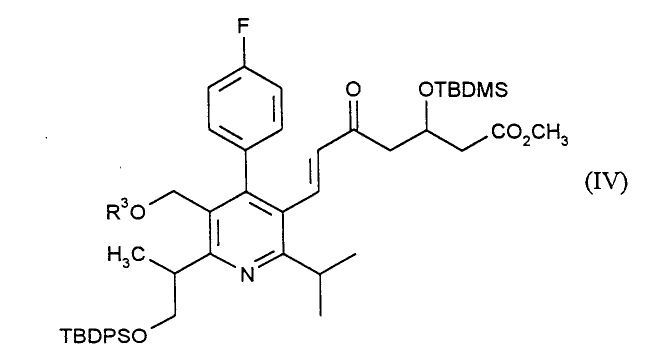 in welcher
- TBDPS: = (CH₃)₃C(C₆H₅)₂Si bedeutet,
und
- R³: die angegebene Bedeutung hat,
herstellt,
diese anschließend durch Abspaltung der Hydroxyschutzgruppen TBPS in die Verbindungen der allgemeinen Formel (V) 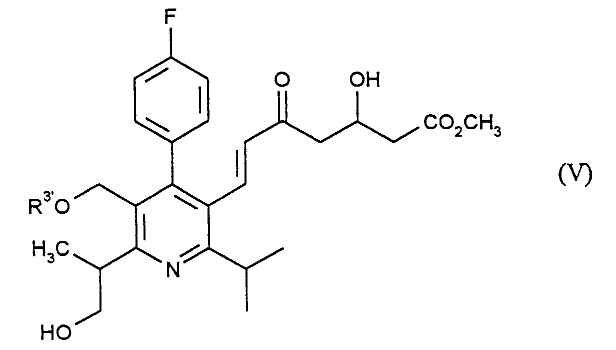 in welcher
- R^{3'}: die angegebene Bedeutung hat
überführt
und in einem letzten Schritt in inerten Lösemitteln mit Natriumborhydrid / Triethylboran die Ketogruppe reduziert,
und im Fall der Säuren die Ester hydrolysiert,
und gegebenenfalls Gemische von Diastereomeren durch Chromatographie oder Kristallisation trennt und in die enantiomerenreinen Verbindungen überführt.

Das erfindungsgemäße Verfahren kann durch folgendes Formelschema beispielhaft erläutert werden:

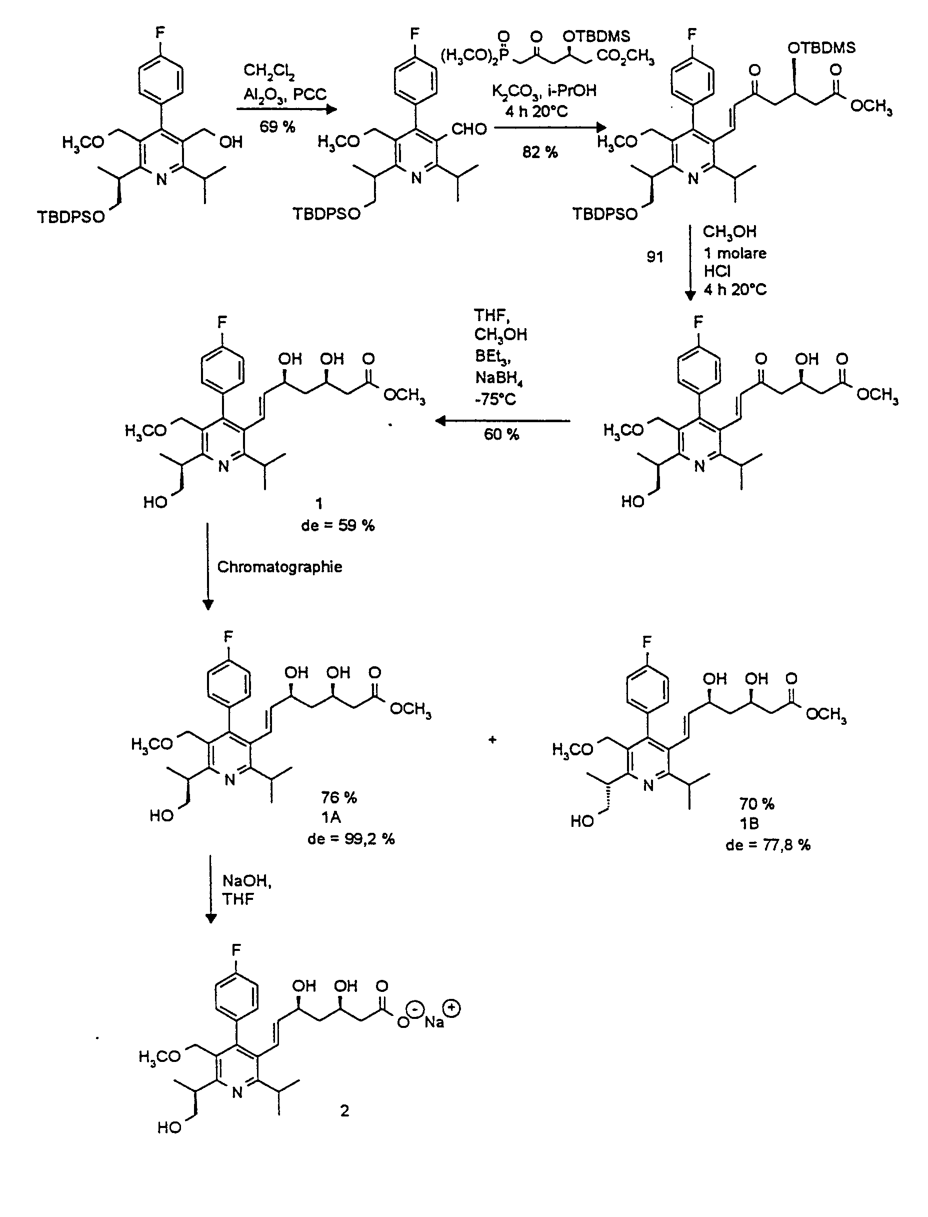

Als Lösemittel für das erfindungsgemäße Verfahren eignen sich Alkohole wie Methanol, Ethanol, Propanol, Isopropanol oder Butanol, oder Ether wie Tetrahydrofuran oder Dioxan, oder Dimethylformamid oder Dimethylsulfoxid, halogenierte Kohlenwasserstoffe, wie beispielsweise Methylenchlorid oder Tetrachlorkohlenstoff oder Wasser. Ebenso ist es möglich, Gemische der genannten Lösemittel einzusetzen.

Bevorzugt sind für die Umsetzung der Verbindungen der allgemeinen Formel (II) Methylenchlorid, für die Herstellung der Verbindungen der allgemeinen Formel (IV) Isopropanol und Wasser, und für die Reduktion der Verbindungen der allgemeinen Formel (V) Tetrahydrofuran und Methanol.

Als Basen eignen sich für die Umsetzung der Verbindungen der Formel (III) Alkali- und Erdalkalicarbonate, wobei Kaliumcarbonat bevorzugt ist.

Die Base wird in einer Menge von 0,5 mol bis 5 mol, bevorzugt von 0,8 mol bis 1,2 mol, bezogen auf 1 mol der Verbindungen der allgemeinen Formel (III) eingesetzt.

Die Abspaltung der Hydroxyschutzgruppen aus den Verbindungen der allgemeinen Formel (IV) erfolgt mit Methanol und Salzsäure.

Alle Umsetzungen des erfindungsgemäßen Verfahrens erfolgen in einem Temperaturbereich von -75°C bis +50°C, vorzugsweise bei Raumtemperatur.

Die Umsetzung erfolgt im allgemeinen bei Normaldruck, es ist jedoch auch möglich, bei Unterdruck oder Überdruck zu arbeiten.

Die Verbindungen der allgemeinen Formel (II) sind neu und können hergestellt werden, indem man im Fall R³ = CH₃

6-[2-(tert.Butyldiphenylsilanyloxy-1-methyl-ethyl]-4-(4-fluorphenyl)-2-isopropyl-1,4-dihydro-pyridin-3,5-dicarbonsäure-dimethylester der Formel (VI) 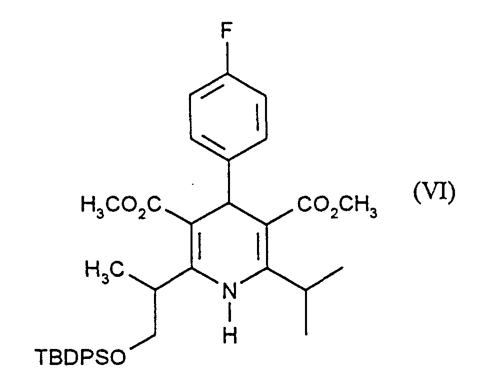 durch Oxidation mit Ammoniumcer(IV)-nitrat in inerten Lösemitteln in das entsprechende Pyridin der Formel (VII) 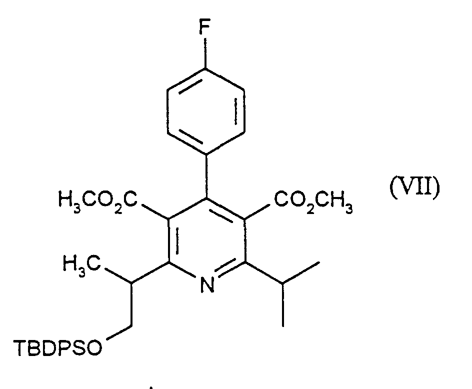 überführt,
anschließend unter Schutzgasatmosphäre mit Diisobutylaluminiumhydrid in inerten Lösemitteln zu den Verbindungen der allgemeinen Formel (VIII) 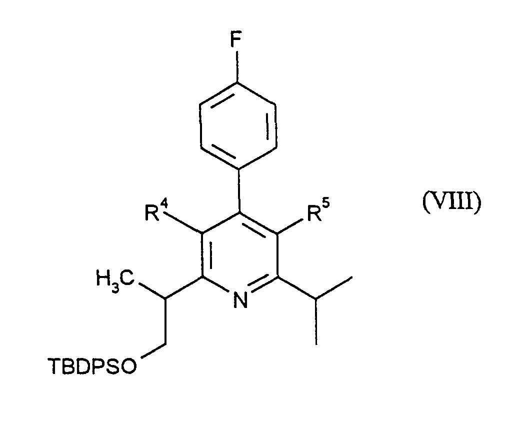 in welcher
- R⁴ und R⁵: jeweils für den Rest der Formel -CO₂CH₃ oder -CH₂OH stehen,
reduziert,
in einem weiteren Schritt durch Umsetzung mit Natriumhydrid in inerten Lösemitteln in die Verbindungen der Formel (IX) 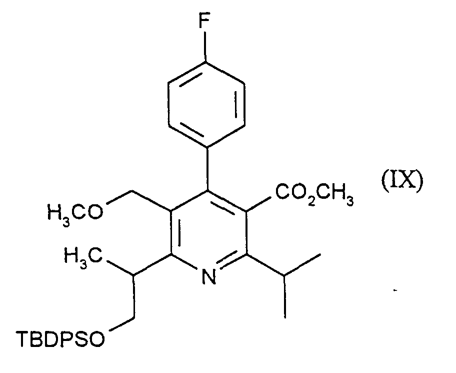 überführt,
und abschließend mit Lithiumaluminiumhydrid in inerten Lösemitteln reduziert.

Als Lösemittel eignen sich für die einzelnen Schritte die oben angegebenen Lösemittel oder Acetonitril, Wasser oder Toluol.

Bevorzugt sind für die Umsetzung der Verbindungen der Formel (VI) Acetonitril/Wasser und für die Herstellungen der Verbindungen der allgemeinen Formeln (VII) und (VIII) Toluol und Tetrahydrofuran.

Die Umsetzungen mit Ausnahme der Reduktionen erfolgen in einem Temperaturbereich von -75°C bis +50°C, vorzugsweise bei Raumtemperatur.

Die Umsetzungen erfolgen im allgemeinen bei Normaldruck, es ist jedoch auch möglich, bei Unterdruck oder Überdruck zu arbeiten.

Als Reduktionsmittel eignen sich für die Umsetzung der Verbindungen der allgemeinen Formel (VII) und die Umsetzung der Verbindungen der allgemeinen Formel (IX) Metallhydride, wie beispielsweise Lithiumaluminiumhydrid, Natriumcyanoborhydrid, Natriumaluminiumhydrid, Diisobutylaluminiumhydrid oder Natrium-bis-(2-methoxyethoxy)dihydroaluminat.

Bevorzugt sind im Fall der Verbindungen der allgemeinen Formel (VII) Diisobutylaluminiumhydrid und im Fall der Verbindungen der allgemeinen Formel (IX) Lithiumaluminiumhydrid.

Das Reduktionsmittel wird im allgemeinen in einer Menge von 4 mol bis 10 mol, bevorzugt von 4 mol bis 5 mol bezogen auf 1 mol der Verbindungen der allgemeinen Formeln (VII) und (VIII) eingesetzt.

Die Reduktion verläuft im allgemeinen in einem Temperaturbereich von -78°C bis +100°C, bevorzugt von -78°C bis 0°C, besonders bevorzugt bei -78°C, jeweils in Abhängigkeit von der Wahl des Reduktionsmittels.

Die Reduktion verläuft im allgemeinen bei Normaldruck, es ist aber auch möglich bei erhöhtem oder erniedrigtem Druck zu arbeiten.

Die Verbindungen der allgemeinen Formel (II), in welchen R³ für den tert.Butyldimethylsilyl-Rest steht, können hergestellt werden, indem man 6-[2-(tert.Butyldiphenylsilanyloxy-1-methyl-ethyl]-4-(4-fluorphenyl)-5-hydroxy-methyl-2-isopropyl-pyridin-3-carbonsäuremethylester in Dimethoxyethan mit tert.Butyldimethylsilylchlorid in Anwesenheit von Imidazol und Dimethylaminopyridin in einem Temperaturbereich von 0°C bis +100°C, vorzugsweise bei +50°C und Normaldruck umsetzt.

Die Verbindungen der Formeln (VII), (VIII) und (IX) sind neu und können wie oben beschrieben hergestellt werden.

Die Verbindung der Formel (VI) ist neu und kann hergestellt werden, indem man 4-Carbomethoxy-5-(4-fluorphenyl)-2-methyl-pent-4-en-3-on mit 5-tert.Butyldiphenylsilanyloxy-4-methyl-3-oxopentansäuremethylester der Formel (X) 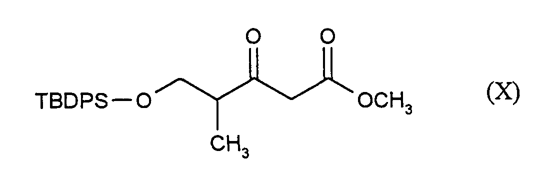 in Methanol/Natriummethylat und Ammoniumacetat/Eisessig bei Raumtemperatur und Normaldruck umsetzt .

Die Verbindung der Formel (X) ist neu und kann hergestellt werden, indem man 3-tert.Butyldiphenylsilanyloxy-2-methylpropionsäuremethylester der Formel (XI) 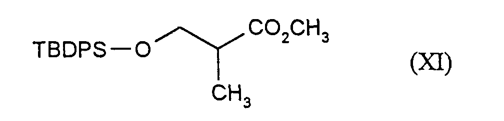 zunächst durch Umsetzung mit Natronlauge in Tetrahydrofuran unter Rückfluß in die entsprechende Säure der Formel (XII) 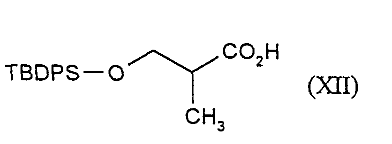 überführt,
anschließend durch Umsetzung mit N,N'-Carbonyldiimidazol in Tetrahydrofuran die Verbindung der Formel (XIII) 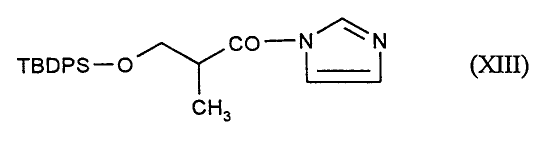 überführt,
und in einem letzten Schritt mit Malonsäuremonomethylester-Kaliumsalz in Acetonitril, Triethylamin und wasserfreiem Magnesiumchlorid in einem Temperaturbereich von 0°C bis +50°C, vorzugsweise von 0°C bis Raumtemperatur und Normaldruck umsetzt.

Die Verbindung der Formel (XI) ist neu und kann hergestellt werden, indem man 3-Hydroxy-2-methylpropionsäuremethylester in Dimethylformamid, tert.Butylchlorodiphenylsilan, Imidazol und 4-Dimethylaminopyridin in einem Temperaturbereich von -10°C bis +60°C, vorzugsweise von 0°C bis +45°C und Normaldruck umsetzt.

Die erfindungsgemäßen Verbindungen besitzen wertvolle, im Vergleich zum Stand der Technik überlegene, pharmakologische Eigenschaften, insbesondere sind sie hochwirksame Inhibitoren der 3-Hydroxy-3-methyl-glutarylCoenzymA(HMG-CoA)Reduktase und infolge dessen Inhibitoren der Cholesterolbiosynthese. Sie können deshalb zur Behandlung von Hyperlipoproteinämie oder Arteriosklerose eingesetzt werden. Die erfindungsgemäßen Wirkstoffe bewirken eine Senkung des Cholesteringehaltes im Blut.

Die pharmakologische Wirkungen der erfindungsgemäßen Stoffe wurden in folgendem Test bestimmt:

### Biologischer Test für HMGCoA-Reduktaseinhibitoren

Cholesterin wird im Säugetierorganismus aus Acetateinheiten aufgebaut. Um die hepatische Cholesterinbiosynthese in vivo zu messen, wurde den Tieren radioaktiv markiertes ¹⁴C-Acetat appliziert und später der Gehalt an ¹⁴C-Cholesterin in der Leber bestimmt.

Die zu untersuchenden Substanzen wurden auf eine Hemmung der hepatischen Cholesterinbiosynthese in vivo an männlichen Wistar-Ratten mit einem Körpergewicht zwischen 140 und 160g geprüft. Die Ratten wurden zu diesem Zweck 18 Stunden vor der oralen Applikation der Substanzen gewogen, in Gruppen zu 6 Tieren (Kontrollgruppe ohne Substanzbelastung 8 Tiere) eingeteilt und nüchterngesetzt. Die zu untersuchenden Substanzen wurden direkt vor der Applikation in wäßriger 0,75%iger Traganthsuspension mit einem Ultra-Turrax suspendiert. Die Applikation der Traganthsuspension (Kontrolltiere) bzw. der in Traganth suspendierten Substanzen erfolgte mittels einer Schlundsonde. 2 Stunden nach der oralen Substanzgabe wurde den Tieren ¹⁴C-Acetat (12,5 µCi/Tier) intraperitoneal injiziert.

Weitere 2 Stunden später (4 Stunden nach Substanzapplikation wurden die Tiere durch Halsschnitt getötet und entblutet. Anschließend wurde der Bauchraum geöffnet und eine Leberprobe von ca. 700mg zur Bestimmung des aus ¹⁴C-Acetat entstandenen ¹⁴C-Cholesterins entnommen. Die Extraktion des Cholesterins erfolgte modifiziert nach Duncan et al. (J. Chromatogr. 162 (1979) 281-292). Die Leberprobe wurde in einem Glaspotter in Isopropanol homogenisiert. Nach Schütteln und anschließender Zentrifugation wurde der Überstand mit alkoholischer KOH versetzt und die Cholesterinester verseift. Nach der Verseifung wurde das Gesamtcholesterin mit Hexan ausgeschüttelt und der Überstand eingedampft. Der Rückstand wurde in Isopropanol aufgenommen, in Szintillationsröhrchen überführt und mit LSC-Cocktail aufgefüllt. Das aus ¹⁴C-Acetat in der Leber synthetisierte ¹⁴C-Cholesterin wurde im Flüssigkeitsszintillationszähler gemessen. Der hepatische ¹⁴C-Cholesteringehalt, der nur mit Traganth behandelten Tiere diente als Kontrolle. Die inhibitorische Aktivität der Substanzen wird in % des synthetisierten hepatischen ¹⁴C-Cholesteringehaltes der Traganth-Kontrolltiere (=100%) angegeben.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5Gew.-%, bevorzugt von 0,5 bis 95Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,1µg/kg bis etwa 100µg/kg, bevorzugt in Gesamtmengen von etwa 1µg/kg bis 50µg/kg Körpergewicht je 24Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und dem Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Experimenteller Teil

### Beispiel I:

### (R)-3-tert.-Butyldiphenylsilanyloxy-2-methylpropionsäuremethylester

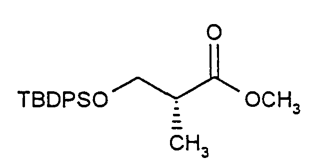

In einem 40-l-Rührwerkskessel werden 2667,0 g (22,60 mol) (R)-(-)-3-Hydroxy-2-methylpropionsäuremethylester (EGA) in 14 1 DMF p.a. gelöst. Nach Zugabe von 6768,8 g (24,65 mol) tert.-Butylchlorodiphenylsilan, 3376,4 g (49,65 mol) Imidazol und 10 g 4-Dimethylaminopyridin steigt die Reaktionstemperatur auf 45°C. Das Reaktionsgemisch wird unter Kühlung bei Raumtemperatur 16 Stunden bis zum vollständigen Umsatz gerührt. Es wird auf 75 l Wasser gegeben, zweimal mit je 20 l Essigester gewaschen, die vereinigten organischen Phasen zweimal mit je 10 l Wasser gewaschen, über Natriumsulfat getrocknet und zu einem Öl einrotiert.

| | |
|---|---|
| Rohausbeute | 8928 g; 110 % d. Th. |
| HPLC | 89,93 % |
| DC | R_{f} = 0,78 (Petrolether/Essigester 9:1) |

¹H-NMR (200 MHz, d₆-DMSO, TMS): δ = 0,98 (s, 9H, t-Bu); 1,10 (d, 3H, CH₃); 2,74 (m, 1H, CH); 3,64 (s, 3H, OCH₃); 3,78 (d, 2H, OCH₂); 7,47 (m, 6H, Ar); 7,61 (m, 4H, Ar) ppm.

### Beispiel II:

### (R)-3-tert.-Butyldiphenylsilanyloxy-2-methylpropionsäure

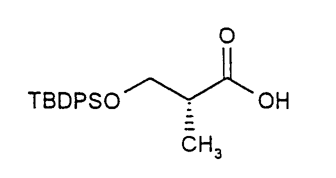

In einem 40-l-Rührwerkskessel wird eine Lösung von 4464 g (11,3 mol) der Verbindung aus Beispiel I (Rohprodukt) in 27,5 1 THF mit 5,65 l (11,3 mol) 2 molarer Natronlauge 46 Stunden unter Rückfluß (65°C Innentemperatur) erhitzt. Am Rotationsverdampfer wird THF abdestilliert, der Rückstand mit 5 l Wasser und 3 l Dichlormethan verdünnt und mit 15 %iger Salzsäure auf pH 4 eingestellt. Die Phasen werden getrennt, die wässrige Phase mit 3 l Dichlormethan gewaschen, die vereinigten organischen Phasen über Natriumsulfat getrocknet und zu einem Öl eingeengt.

| | |
|---|---|
| Rohausbeute | 3930 g; 100 % d. Th. |
| HPLC | 67,17 % |
| | 14,21 % Silylnebenprodukt |
| | 16,81 % Edukt I |
| DC | R_{f} = 0,27 (Petrolether/Essigester 9:1) |

¹H-NMR (200 MHz, d₆-DMSO, TMS): δ = 1,00 (s, 9H, t-Bu); 1,08 (d, 3H, CH₃); 2,60 (m, 1H, CH); 3,74 (m, 2H, OCH₂); 7,43 (m, 6H, Ar); 7,61 (m, 4H, Ar); 12,26 (s, 1H, COOH) ppm.

### Beispiel III:

### (R)-3-tert.-Butyldiphenylsilanyloxy-2-methylpropionsäure-imidazolid

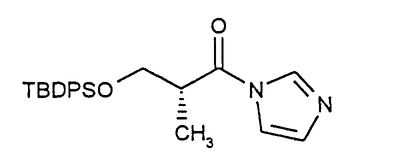

Zu einer Lösung von 1927,5 g (3,77 mol) der Verbindung aus Beispiel II (67 %ig) in 13 1 THF werden bei Raumtemperatur 744,4 g (4,59 mol) N,N'-Carbonyldiimidazol gegeben. Das Reaktionsgemisch wird 1 Stunde bei Raumtemperatur und 1 Stunde unter Rückfluß gerührt. Nach dem Abkühlen auf Raumtemperatur wird die Lösung ohne weitere Reinigung in die nächste Stufe eingesetzt.

### Beispiel IV:

### (R)-5-tert.-Butyldiphenylsilanyloxy-4-methyl-3-oxopentansäuremethylester

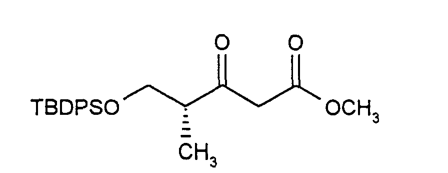

In einem 40-1-Rührwerkskessel werden 1258,3 g (8,06 mol) Malonsäuremonomethylester-Kaliumsalz in 12,4 l Acetonitril bei 0°C suspendiert. Es werden 1124,5 ml (8,06 mol) Triethylamin und 847,1 g (8,92 mol) wasserfreies Magnesiumchlorid zugegeben und 5 Stunden bei Raumtemperatur gerührt. Innerhalb 15 min werden die Reaktionslösung III und 112,4 ml (0,81 mol) Triethylamin zugegeben, anschließend wird das Reaktionsgemisch 16 Stunden bei Raumtemperatur gerührt. Es wird mit 20 l Essigester verdünnt und mit 15 %iger Salzsäure auf pH 4 eingestellt. Die organische Phase wird abgetrennt, mit 10 1 Wasser gewaschen, eingeengt, in 20 l Essigester aufgenommen und restliches Wasser abgetrennt. Es wird zweimal mit je 10 l gesättigter Natriumhydrogencarbonatlösung neutral gewaschen, über Natriumsulfat getrocknet und zu einem Öl einrotiert.

| | |
|---|---|
| Rohausbeute | 2012 g; 84,7 % d. Th. |
| HPLC | 63,24 % |
| | 14,57 % Silylnebenverbindung |
| | 17,30 % Edukt I |
| DC | R_{f} = 0,56 (Petrolether/Essigester 9:1) |

¹H-NMR (200 MHz, d₆-DMSO, TMS): δ = 1,00 (s, 9H, t-Bu und d, 3H, CH₃); 2,95 (m, 1H, CH); 3,65 (s, 3H, OCH₃); 3,72 (m, 4H, CH₂, OCH₂); 7,40 (m, 6H, Ar); 7,61 (m, 4H, Ar) ppm.

### Beispiel V:

### (E/Z)-4-Carbomethoxy-5-(4-fluorphenyl)-2-methyl-pent-4-en-3-on

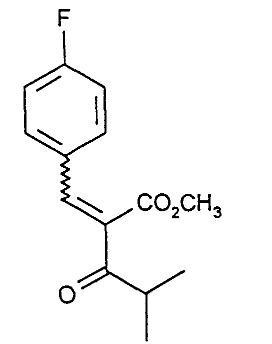

In 1,4 l Cyclohexan werden 2094 g (14,54 mol) Isobutyryl-essigsäuremethylester, 1442 g (11,6 mol) 4-Fluorbenzaldehyd, 46 ml Eisessig und 81 ml Piperidin gelöst und am Wasserabscheider unter Rückfluß erhitzt. Innerhalb 2,5 Stunden scheiden sich 240 ml Wasser ab. Bei 80 mbar werden Cyclohexan und Eisessig abdestilliert, anschließend werden bei 2 mbar (Badtemperatur 120°C, Kopftemperatur 70°C) die Edukte abdestilliert. Der Rückstand wird bei Raumtemperatur mit 3 l Essigester versetzt, mit Natriumhydrogencarbonat gewaschen, über Natriumsulfat getrocknet und zu einem Öl eingeengt.

| | |
|---|---|
| Rohausbeute | 2930 g; 61,14 % d. Th. |
| HPLC | 31,51 % |
| | 61,14 % E/Z |

¹H-NMR (200 MHz, CDCl₃, TMS): δ = 1,10 und 1,18 (2d, 6H, CH₃); 2,71 und 3,18 (2 sept., 1H, CH); 3,84 (2s, 3H, OCH₃); 7,07 (m, 2H, Ar); 7,40 (m, 2H, Ar); 7,58 und 7,75 (2s, 1H, OlefinH) ppm.
Durch Verreiben mit Petrolether wird ein Diastereomer als Feststoff erhalten.

| | |
|---|---|
| Schmelzpunkt | 56 - 58°C |

¹H-NMR (CDCl₃): δ = 1,09 (d, 6H, CH₃); 2,71 (sept., 1H, CH); 3,84 (s, 3H, OCH₃); 7,07 (m, 2H, Ar); 7,40 (m, 2H, Ar); 7,75 (s, 1H, OlefinH) ppm.

### Beispiel VI:

### 6-[2-(tert.-Butyldiphenylsilanyloxy-1(S)-methyl-ethyl]-4(R,S)-(4-fluorphenyl)-2-isopropyl-1,4-dihydro-pyridin-3,5-dicarbonsäure-dimethylester

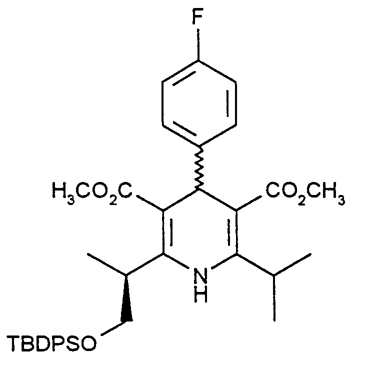

Eine Lösung von 560 g (2,01 mol) der Verbindung aus Beispiel V (Rohprodukt, 92 %ig) und 1560 g (2,01 mol) der Verbindung aus Beispiel IV (Rohprodukt, 50 %ig) in 3 l Methanol wird auf 10°C gekühlt und portionsweise mit 104 g (1,92 mol) Natriummethylat versetzt, wobei eine leichte Erwärmung stattfindet. Es wird bis zum vollständigen Umsatz der Verbindung aus Beispiel V bei Raumtemperatur gerührt (1,5 Stunden, HPLC-Kontrolle). Anschließend werden 480 g (6,25 mol) Ammoniumacetat sowie 1,65 1 Eisessig hinzugegeben und bei einer Badtemperatur von 130 - 140°C flüchtige Anteile über eine Destillationsbrücke abdestilliert (Innentemperatur 112°C, Kopftemperatur 105°C). Nach 90 min ist die Michael-Additionsverbindung vollständig umgesetzt. Bei Raumtemperatur werden 3 1 Wasser zugegeben. Es wird dreimal mit je 1,5 l Essigester gewaschen, die vereinigten organischen Phasen mit 2 l Wasser und 2 1 Natriumhydrogencarbonatlösung gewaschen, über Natriumsulfat getrocknet und zu einem Öl eingeengt.

| | |
|---|---|
| Rohausbeute | 2015 g; 72,1 % d. Th. bezogen auf 45,4 % Gehalt |
| HPLC | 20,68 % und 24,73 % (2 Diastereomere) |

¹H-NMR (CDCl₃): δ = 0,94 - 1,35 (mehrere d, 9H, CH₃); 1,13 (mehrere s, 9H, t-Bu); 3,59, 3,63, 3,64, 3,67 (4s, 6H, 2 OCH₃); 3,73 - 4,30 (komplexer Bereich, 4H, CH, OCH₂); 4,99 und 5,03 (2s, 1H, DHP-H); 6,80 - 7,78 (komplexer Bereich, 14H, Ar) ppm.
- FAB-MS:: m/z = 630(M+H)⁺, 598, 534, 374, 322, 278, 213, 199, 197, 183, 135.

### Beispiel VII:

### 6-[2-(tert.-Butyldiphenylsilanyloxy-1(S)-methyl-ethyl]-4-(4-fluorphenyl)-2-isopropyl-pyridin-3,5-dicarbonsäure-dimethylester

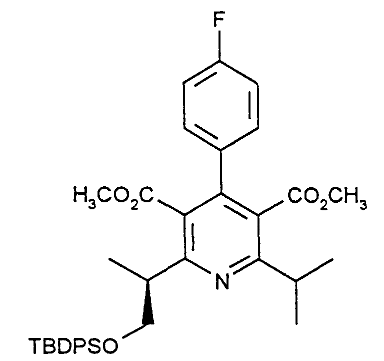

In 10 l Acetonitril werden 2015 g (ca 1,45 mol) der Verbindung aus Beispiel VI gelöst. Unter Rühren werden bei Raumtemperatur 1698 g (3,1 mol) Ammoniumcer-(IV)-nitrat portionsweise innerhalb 10 min zugegeben, wobei sich das Reaktionsgemisch (Suspension) auf ca. 34°C erwärmt. Nach 30 min ist der Umsatz vollständig (HPLC-Kontrolle). Das Reaktionsgemisch wird mit 12 1 Wasser verrührt und das Acetonitril aus dem Zweiphasengemisch am Rotationsverdampfer weitgehend abdestilliert. Wegen der Explosionsgefahr sollte beim Einengen stets Wasser vorhanden sein. Der wässrige Rückstand wird dreimal mit je 3 l Essigester gewaschen, die vereinigten organischen Phasen mit 3 l 10 %iger Kaliumiodidlösung, mit 3 l Natriumthiosulfatlösung und mit 5 l Wasser gewaschen, über Natriumsulfat getrocknet und zu 1869 g Rohöl eingeengt. Das Öl wird über 12 kg Kieselgel 60 mit ca. 90 l Petrolether/Essigester 97:3 chromatographiert.

| | |
|---|---|
| Ausbeute | 577,4 g Öl; 36,1 % d. Th. |
| HPLC | 57,29 % |

Eine Reinprobe (Öl) wird durch nochmalige Chromatographie erhalten.
¹H-NMR (200 MHz, CDCl₃, TMS): δ = 0,96 (s, 9H, t-Bu); 1,25 (dd, 9H, CH₃); 3,07 (sept., 1H, CH(CH₃)₂; 3,30 (m, 1H, CH-CH₂); 3,48 (s, 3H, OCH₃); 3,57 (s, 3H, OCH₃); 3,70 und 4,05 (m, je 1H, CH₂O); 7,00 - 7,65 (m, 14 H, Ar) ppm.

### Beispiele VIII, IX und X:

### 6-[2-(tert.-Butyldiphenylsilanyloxy-1(S)-methyl-ethyl]-4-(4-fluorphenyl)-5-hydroxymethyl-2-isopropyl-pyridin-3-carbonsäuremethylester

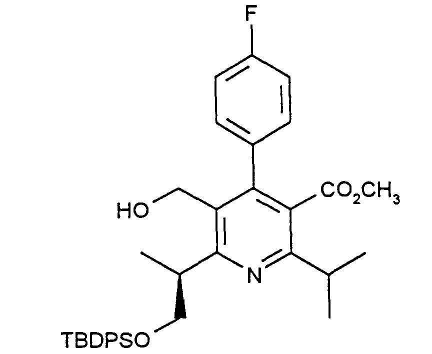

Eine Lösung von 561 g (0,51 mol) der Verbindung aus Beispiel VII (57,29 %ig) in 1,7 l Toluol p.a. wird unter Argonatmosphäre auf -60°C gekühlt. Zu dieser Lösung werden innerhalb 1,5 Stunden 1367 ml (2,05 mol) einer 1,5 molaren Diisobutylaluminiumhydridlösung in Toluol so zugetropft, daß die Innentemperatur -53°C nicht übersteigt. Nach beendeter Zugabe wird 30 min bei -60°C und 16 Stunden bei -30°C bis zum fast vollständigen Umsatz gerührt (DC-Kontrolle: Petrolether/Essigester 9:1). Zur Hydrolyse der Aluminiumverbindungen wird das Reaktionsgemisch innerhalb 20 min unter Rühren zu 8 l 10 %iger Kaliumhydroxidlösung gegeben, 15 min nachgerührt, die wässrige Phase abgetrennt, zweimal mit je 3 l Essigester gewaschen und zum Abtrennen von Ausflockungen, die die Phasentrennung erschweren, durch Kieselgur gesaugt. Die vereinigten organischen Phasen werden zweimal mit je 2 1 gesättigter Kochsalzlösung gewaschen, über Natriumsulfat getrocknet und zu 525 g Rohöl am Rotationsverdampfer eingeengt. Das Rohöl wird über 13 kg Kieselgel 60 mit 100 1 Petrolether/Essigester 9:1 und 40 l Petrolether/Essigester 8:2 chromatographiert.

| | |
|---|---|
| Ausbeute | 124,9 g Öl; 43,2 % d. Th. |
| HPLC | 99,0 % |
| DC | R_{f} = 0,36 (Petrolether/Essigester 9:1) |

¹H-NMR (CDCl₃): δ = 0,95 (s, 9H, t-Bu); 1,17 (d, 3H, CH₃); 1,25 (2d, 6H, CH₃); 3,05 (sept., 1H, CH); 3,45 (m, 1H, CH-CH₂); 3,57 (s, 3H, OCH₃); 3,62 - 3,77 (m, 2H, CH₂O); 4,30 und 4,61 (m, je 1H, CH₂OSi); 7,02 - 7,65 (m, 14H, Ar) ppm.
- FAB-MS:: m/z = 600(M+H)⁺, 542, 344, 326, 312, 199, 137, 135.

Weiterhin werden 42,3 g Edukt VII (HPLC: 99,26 %; 13,2 % d. Th.) und 244,3 g (HPLC: 34,95 %; 29,5 % d. Th.) 6-[2-(tert.-Butyldiphenylsilanyloxy-1(S)-methylethyl]-4-(4-fluorphenyl)-3-hydroxymethyl-2-isopropyl-pyridin-5-carbonsäuremethylester 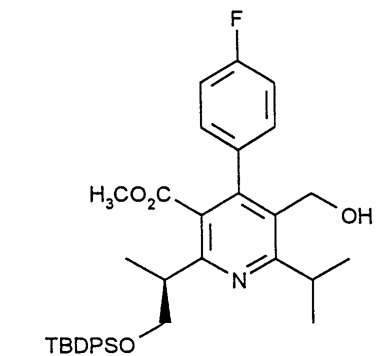 isoliert. Durch nochmalige Chromatographie wird eine Reinprobe (Öl) erhalten. ¹H-NMR (200 MHz, CDCl₃, TMS): δ = 0,95 (s, 9H, t-Bu); 1,23 - 1,35 (mehrere d, 9H, CH₃); 3,22 (ddq, 1H, CH); 3,45 (s, 3H, OCH₃); 3,48 (sept., 1H, CH); 3,81 und 4,08 (m, 2H, OCH₂Si); 4,45 (2d, 2H, OCH₂); 7,02 - 7,67 (m, 14H, Ar) ppm.

In Vorversuchen wurde 6-[2-(tert.-Butyldiphenylsilanyloxy-1(S)-methyl-ethyl]-4-(4-fluorphenyl)-3,5-bishydroxymethyl-2-isopropyl-pyridin 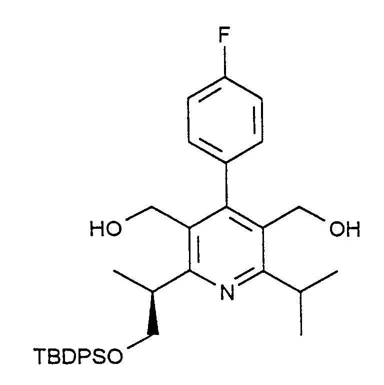 erhalten.

| | |
|---|---|
| Schmelzpunkt | 136°C |
| DC | R_{f} = 0,12 (Petrolether/Essigester 9:1) |

¹H-NMR (200 MHz, CDCl₃, TMS): δ = 0,91 (s, 9H, t-Bu); 1,20 - 1,35 (3d, 9H, CH₃); 1,38 (tr, 2H, OH); 3,28 (m, 1H,); 3,49 (sept., 1H, CH); 3,67 (m, 1H); 3,80 (m, 2H); 4,13 und 4,38 (2m, 2H); 4,55 (m, 2H); 7,08 - 7,61 (komplexer Bereich, 14H, Ar) ppm.

| | |
|---|---|
| FAB-MS | m/z = 572 (M+H)⁺, 598, 534, 374, 322, 278, 213, 199, 197, 183, 135. |
| ee = | 59,4 % (HPLC) |

### Beispiel XI:

### 6-[2-(tert.-Butyldiphenylsilanyloxy-1(S)-methyl-ethyl]-4-(4-fluorphenyl)-2-isopropyl-5-methoxymethyl-pyridin-3-carbonsäuremethylester

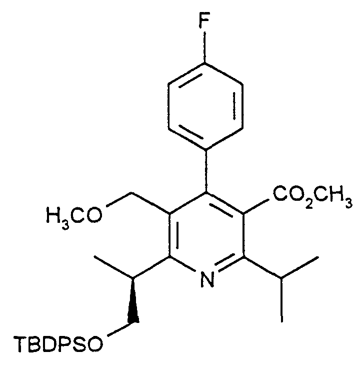

9,35 g (0,313 mol) Natriumhydrid (80 %ig) werden in 500 ml absolutem THF suspendiert und zum Sieden erhitzt. Unter Rückfluß wird eine Lösung von 125 g (0,209 mol) der Verbindung aus Beispiel VIII in 300 ml absolutem THF zugetropft. Dann wird ebenfalls unter Rückfluß eine Lösung von 35,5 g (0,25 mol) Methyliodid in 100 ml absolutem THF zugetropft. Anschließend wird weitere 3 Stunden unter Rückfluß erhitzt. Nach Abkühlen auf Raumtemperatur werden vorsichtig 250 ml Wasser zugegeben. Dann wird dreimal mit je 300 ml Essigester extrahiert, die vereinigten organischen Phasen mit gesättigter Kochsalzlösung gewaschen und über Natriumsulfat getrocknet. Nach Abziehen des Lösungsmittels im Vakuum wird der Rückstand über Kieselgel chromatographiert (1 kg Kieselgel 60, Laufmittel Petrolether/Essigester 95:5).

| | |
|---|---|
| Ausbeute | 117,37 g; 91,74 % d. Th. |
| DC | R_{f} = 0,55 (Petrolether/Essigester 9:1) |

¹H-NMR (CDCl₃): δ = 0,95 (s, 9H, t-Bu); 1,22 (2d, 6H, CH₃); 1,30 (d, 3H, CH₃); 3,05 (sept., 1H, CH); 3,20 (s, 3H, OCH₃); 3,51 (s, 3H, OCH₃); 3,58 (m, 1H, CH-CH₂); 3,8 - 4,0 (m, 2H, CH₂O und 1H CH₂OSi); 4,45 (dd, 1H, CH₂OSi); 7,0 - 7,6 (m, 14H, Ar) ppm.

### Beispiel XII:

### 6-[2-(tert.-Butyldiphenylsilanyloxy-1(S)-methyl-ethyl]-4-(4-fluorphenyl)-2-isopropyl-5-methoxymethyl-pyridin-3-yl]methanol

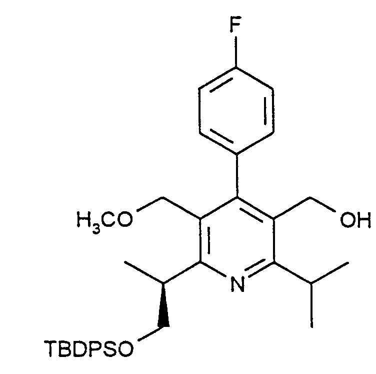

Unter Argon werden 9 g (0,236 mol) Lithiumaluminiumhydrid in 500 ml absolutem THF suspendiert und zum Sieden erhitzt. Unter Rückfluß wird dann eine Lösung von 72,3 g (0,118 mol) der Verbindung aus Beispiel XI in 300 ml absolutem THF zugetropft. Anschließend wird 1 Stunde unter Rückfluß erhitzt. Nach Abkühlen auf Raumtemperatur werden vorsichtig 80 ml Wasser zugetropft. Dann werden 80 ml 10 %iger Kaliumhydroxidlösung zugegeben und der entstehende Niederschlag wird abgesaugt. Der Niederschlag wird dreimal mit je 300 ml Ether ausgekocht. Die Mutterlaugen werden vereinigt, über Natriumsulfat getrocknet und dann am Vakuum eingeengt. Das so erhaltene Rohprodukt wird ohne weitere Reinigung in die nächste Stufe eingesetzt.

| | |
|---|---|
| Ausbeute | 69 g; 99 % d. Th. |
| DC | R_{f} = 0,25 (Petrolether/Essigester 9:1) |

¹H-NMR (CDCl₃): δ = 0,95 (s, 9H, t-Bu); 1,25 (2d, 6H, CH₃); 1,22 (d, 3H, CH₃); 3,15 (s, 3H, OCH₃); 3,42 (sept., 1H, CH); 3,53 (m, 1H, CH-CH₃); 3,8 - 4,0 (m, 3H, CH₂O und CH₂OSi); 4,35 - 4,45 (m, 3H, CH₂OH, CH₂OSi); 7,0 - 7,6 (m, 14 H, Ar) ppm.

### Beispiel XIII:

### 6-[2-(tert.-Butyldiphenylsilanyloxy-1(S)-methyl-ethyl]-4-(4-fluorphenyl)-2-isopropyl-5-methoxymethyl-pyridin-3-carbaldehyd

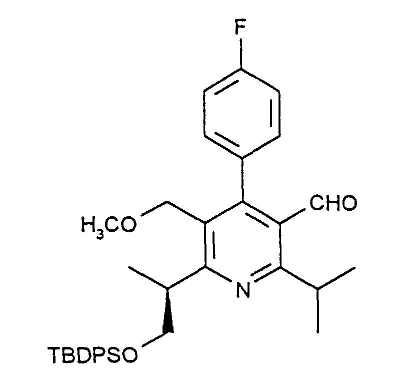

Zu einer Lösung von 138 g (0,236 mol) der Verbindung aus Beispiel XII in 3,5 1 Dichlormethan werden 48,1 g (0,472 mol) Aluminiumoxid und 101,7 g (0,472 mol) Pyridiniumchlorochromat gegeben. Nach 1 Stunde Rühren bei Raumtemperatur wird über eine Fritte mit 500 g Kieselgel 60 und mit ausreichend Dichlormethan nachgewaschen. Anschließend wird im Vakuum eingeengt und getrocknet.

| | |
|---|---|
| Ausbeute | 95,4 g; 69,3 % d. Th. |
| DC | R_{f} = 0,59 (Petrolether/Essigester 9:1) |

¹H-NMR (CDCl₃): δ = 0,9 (s, 9H, t-Bu); 1,22 (d, 6H, 2 x CH₃); 1,30 (d, 3H, CH₃); 3,21 (s, 3H, OCH₃); 3,62 (m, 1H, CH); 3,8 - 4,0 (m, 2H CH₂O, 1H CH-CH₂, 1H CH₂OSi); 4,46 (dd, 1H, CH₂OSi); 7,0 - 7,7 (m, 14H, Ar); 9,78 (s, 1H, CHO) ppm.

### Beispiel XIV:

### Methyl-(E)-7-{6-[2-(tert.-butyldiphenylsilanyloxy)-1(S)-methyl-ethyl]-4-(4-fluorphenyl)-2-isopropyl-5-methoxymethyl-pyrid-3-yl}-(3R)-tert.-butyldimethylsilanyloxy-5-oxo-hept-6-enoat

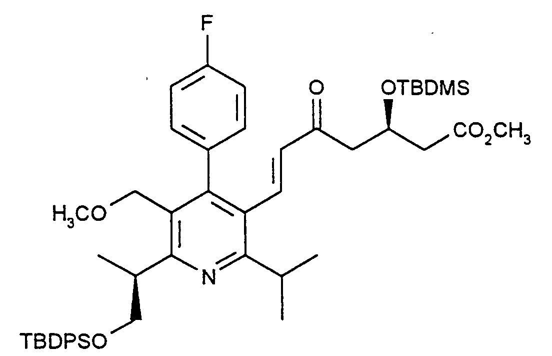

82,5 g (0,22 mol) R-Ketophosphonat (CH₃O)₂PO-CH₂-CO-CH₂CH(OTBDMS)-CH₂-CO₂CH₃, 29,6 g (0,214 mol) Kaliumcarbonat und 2,9 ml Wasser werden in 635 ml Isopropanol gelöst und 1 Stunde bei Raumtemperatur gerührt. Anschließend werden 95,4 g (0,16 mol) der Verbindung aus Beispiel XIII suspendiert in 150 ml Isopropanol zugegeben. Nach 4 Tagen Rühren bei Raumtemperatur (DC-Kontrolle) werden 500 ml Wasser zugegeben und anschließend wird dreimal mit 500 ml Essigester ausgeschüttelt. Die vereinigten Essigesterphasen werden mit gesättigter Kochsalzlösung gewaschen, über Natriumsulfat getrocknet und im Vakuum eingeengt. Der Rückstand wird über Kieselgel chromatographiert (1 kg Kieselgel 60, Laufmittel Petrolether/Essigester 95:5).

| | |
|---|---|
| Ausbeute | 112,1 g; 81,6 % d. Th. |
| DC | R_{f} = 0,32 (Petrolether/Essigester 9:1) |

¹H-NMR (CDCl₃): -0,2 (s, 3H, CH₃Si); 0,3 (s, 3H, CH₃Si); 0,75 (s, 9H, t-Bu); 0,9 (s, 9H, t-Bu); 1,15 - 1,35 (m, 9H, 3 x CH₃); 2,38 (m, 2H, CH₂); 2,56 (d, 2H, CH₂); 3,12 (s, 3H, OCH₃); 3,23 (sept., 1H, CH); 3,52 (m, 1H, CH-CH₂); 3,61 (s, 3H, OCH₃); 3,75 - 3,95 (m, 2H CH₂O und 1H CH₂OSi); 4,38 (dd, 1H, CH₂OSi); 4,47 (m, 1H, CHOSi); 5,85 (d, 1H, =CH); 6,9 - 7,6 (m, 14H, Ar und 1H =CH) ppm.

### Beispiel XV:

### Methyl-(E)-7-{4-(4-fluorphenyl)-6-[1(S)-hydroxymethyl-ethyl]-2-isopropyl-5-methoxymethyl-pyrid-3-yl}-(3R)-hydroxy-5-oxo-hept-6-enoat

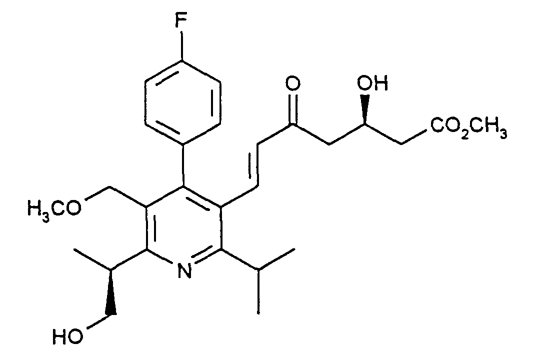

Eine Lösung von 112 g (0,133 mol) der Verbindung aus Beispiel XIV in 1170 ml absolutem Methanol und 130 ml 1 molarer Salzsäure wird 4 Tage bei Raumtemperatur gerührt (DC-Kontrolle). Anschließend werden 1000 ml Dichlormethan zugegeben und zweimal mit je 500 ml gesättigter Natriumhydrogencarbonatlösung extrahiert. Die organische Phase wird mit Natriumsulfat getrocknet und im Vakuum eingeengt. Der Rückstand wird über Kieselgel chromatographiert (1 kg Kieselgel 60, Laufmittel Petrolether/Essigester 1:1).

| | |
|---|---|
| Ausbeute | 59,2 g; 91 % d. Th. |
| DC | R_{f} = 0,17 (Petrolether/Essigester 1:1) |

¹H-NMR (CDCl₃): δ = 1,28 (2d, 6H, CH₃); 1,42 (d, 3H, CH₃); 2,48 (m, 2H, CH₂); 2,61 (m, 2H, CH₂); 3,20 (s, 3H, OCH₃); 3,28 (sept., 1H, CH); 3,32 (m, 2H, CH₂OH); 3,71 (s, 3H, OCH₃); 3,88 (m, 1H, CH-CH₂); 4,0 - 4,2 (m, 2H, CH₂O); 4,41 (m, 1H, CHOH); 5,90 (d, 1H, =CH); 7,0 - 7,2 (m, 4H, Ar); 7,45 (d, 1H, =CH) ppm.

### Beispiel XVI:

### 6-[2-(tert.-Butyldiphenylsilanyloxy)-1(S)-methyl-ethyl]-5-tert-butyldimethylsilanyloxymethyl-4-(4-fluorphenyl)-2-isopropyl-pyridin-3-carbonsäuremethylester

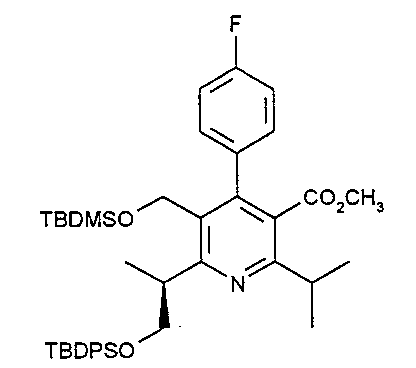

3 g (0,05 mol) der Verbindung aus Beispiel VIII werden in 400 ml Dimethoxyethan mit 16,6 g (0,11 mol) tert-Butyldimethylsilylchlorid, 15 g (0,22 mol) Imidazol und 1 g (8 mmol) Dimethylaminopyridin über Nacht bei 50° C gerührt. Nach dem Abkühlen wird mit 100 ml Diethylether verdünnt und mit 200 ml gesättigter Kochsalzlösung extrahiert. Die organische Phase wird nach dem Trocknen mit Natriumsulfat eingeengt und der Rückstand über Kieselgel chromatographiert (500 g Kieselgel 60, Laufmittel Petrolether/Essigester 95:5)

| | |
|---|---|
| Ausbeute | 35,57 g; 99,8% der Theorie. |
| DC | R_{f} = 0,30 (Petrolether/Essigester 95:5) |

¹H-NMR (CDCl₃): δ = -0,3 (s, 3H); 0,1 (s, 3H); 0,87 und 0,92 (2s, 18H); 1,2 - 1,35 (3d, 9H); 3,0 (sept, 1H); 3,51 (s, 3H); 3,6 - 3,75 (m, 1H); 3,8 - 4,25 (m, 4 H); 4,78 (dd, 1H); 6,95 - 7,65 (m, 14 H) ppm.

### Beispiel XVII:

### 6-[2-(tert.-Butyldiphenylsilanyloxy)-1(S)-methyl-ethyl]-5-tert.-butyldimethylsilanyloxymethyl-4-(4-fluorphenyl)-3-hydroxymethyl-2-isopropylpyridin

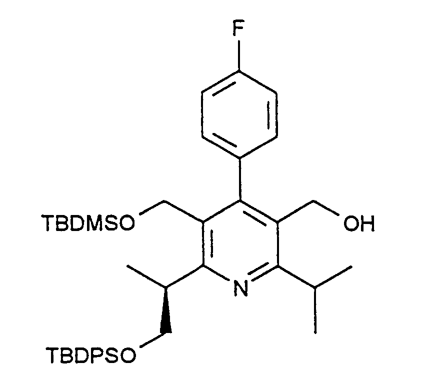

Unter Argon werden 3,79 g (0,1 mol) Lithiumaluminiumhydrid in 250 ml absolutem THF suspendiert und zum Sieden erhitzt. Unter Rückfluß wird eine Lösung von 35,57 g (0,05 mol) der Verbindung aus Beispiel XVI in 150 ml absolutem THF zugetropft. Anschließend wird 1 Stunde unter Rückfluß erhitzt. Nach dem Abkühlen auf Raumtemperatur werden vorsichtig 30 ml Wasser zugetropft. Dann werden 30 ml 10%ige Kaliumhydroxid-Lösung zugegeben und der entstehende Niederschlag abgesaugt. Der Niederschlag wird dreimal mit je 100 ml Diethylether ausgekocht. Die Mutterlaugen werden vereinigt, über Natriumsulfat getrocknet und dann im Vakuum eingeengt. Das so erhaltene Rohprodukt wird ohne weitere Reinigung in die nächste Stufe eingesetzt.
- Ausbeute:: 33,49 g; 98% der Theorie.
- DC:: R_{f} = 0,24 (Petrolether : Essigester 9 : 1)
¹H-NMR (CDCl₃) δ = -0,8 (s, 3H); -0,2 (s, 3H); 0,85 und 0,92 (2s, 18H); 1,2 - 1,35 (3d, 9H); 3,42 (sept, 1H); 3,6 - 4,15 (m, 6H); 4,4 (AB, 2H); 4,72 (d, 1H); 6,95 - 7,65 (m, 14H) ppm.

### Beispiel XVIII:

### 6-[2-(tert.-Butyldiphenylsilanyloxy)-1(S)-methyl-ethyl]-5-tert.-butyldimethylsilanyloxymethyl-4-(4-fluorphenyl)-2-isopropyl-pyridin-3-carbaldehyd

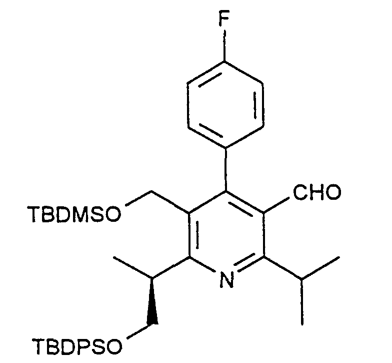

Zu einer Lösung von 33,49 g (0,1 mol) der Verbindung aus Beispiel XVII in 600 ml Dichlormethan werden 9,97 g Aluminiumoxid und 21,08 g (0,098 mol) Pyridiniumchlorochromat gegeben. Nach 1 Stunde Rühren bei Raumtemperatur wird über eine Fritte mit Kieselgel (100 g Kieselgel 60) filtriert und mit ausreichend Dichlormethan nachgewaschen. Anschließend wird im Vakuum eingeengt und getrocknet.

| | |
|---|---|
| Ausbeute | 26,56 g; 79,5% der Theorie. |
| DC | R_{f} = 0,20 (Petrolether : Essigester 9 : 1) |

¹H-NMR (CDCl₃) δ = -0,5 (s, 3H); -0,1 (s, 3H); 0,87 und 0,89 (2s, 18H); 1,2 - 1,3 (3d, 9H); 3,65 - 4,2 (m, 2H, CH₂O; 1H, CHCH₂; 1H, CH₂OSi); 4,78 (dd, 1H); 7,05 - 7,65 (m, 14H); 9,78 (s, 1H) ppm.

### Beispiel XIX:

### Methyl-(E)-7-{6-[2-(tert.-Butyldiphenylsilanyloxy)-1(S)-methyl-ethyl]-5-tert.-butyldimethylsilanyl-oxy-methyl-4-(4-fluorphenyl)-2-isopropyl-pyridin-3-yl}-(3R)-tert.-butyldimethylsilanyloxy-5-oxo-hept-6-enoat

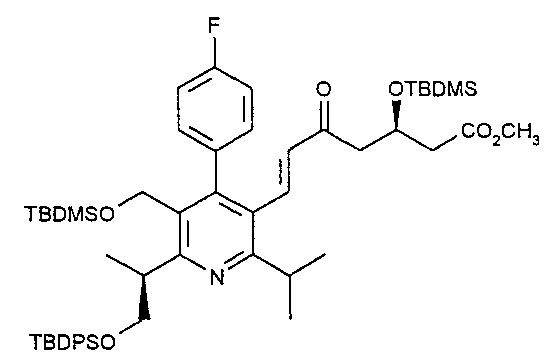

2,4 g (6,38 mmol) R-Ketophosphonat (CH₃O)₂PO-CH₂CH(OTBDMS)-CH₂-CO₂CH₃, 0,875 g (6,33 mmol) Kaliumcarbonat und 85,8 ml H₂O werden in 18,8 ml Isopropanol gelöst und 1 Stunde bei Raumtemperatur gerührt. Anschließend werden 3,3 g (4,83 mmol) der Verbindung aus Beispiel XVIII gelöst in 18,8 ml Isopropanol zugegeben. Nach 5 Tagen Rühren bei Raumtemperatur (DC-Kontrolle) werden 100 ml Wasser zugegeben und anschließend wird dreimal mit 100 ml Essigester ausgeschüttelt. Die vereinigten organischen Phasen werden mit Kochsalzlösung gewaschen, mit Natriumsulfat getrocknet und im Vakuum eingeengt. Der Rückstand wird über Kieselgel chromatographiert (500 g Kieselgel 60, Laufmittel Petrolether : Essigester 95 : 5)

| | |
|---|---|
| Ausbeute | 3,6 g; 79,4% der Theorie. |
| DC | R_{f} = 0,31 (Petrolether : Essigester 9 : 1) |

¹H-NMR (CDCl₃) δ = -0,5, -0,1und 0,2 (3s, 12H); 0,8, 0,87 und 0,9 (3s, 27H); 1,15 - 1,35 (3d, 9H); 2,42 (m, 2H); 2,6 (d, 2H); 3,28 (sept, 1H); 3,65 (s, 3H); 3,8 - 4,2 (m, 3H); 4,52 (m, 1H); 4,73 (d, 1H); 5,90 (d, 1H); 6,9 - 7,65 (m, 15H) ppm.

### Beispiel XX:

### Methyl-(E)-7-{6-[1(S)-hydroxy-methyl-ethyl]-5-hydroxymethyl-4-(4-fluorphenyl)-2-isopropyl-pyridin-3-yl}-(3R)-hydroxy-5-oxo-hept-6-enoat

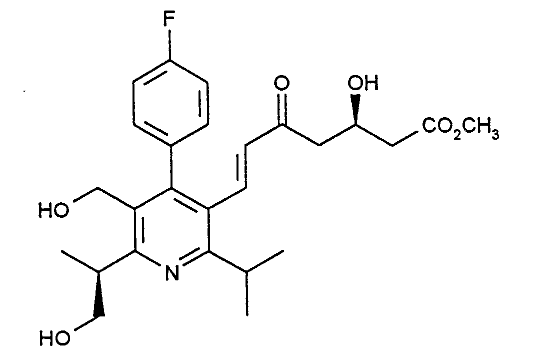

Eine Lösung von 3,6 g (3,8 mmol) der Verbindung aus Beispiel XIX in 63 ml Methanol und 7 ml 1 molarer Salzsäure wird 5 Tage bei Raumtemperatur gerührt. Anschließend werden 100 ml Dichlormethan zugegeben und es wird zweimal mit je 100 ml gesättigter Natriumhydrogencarbonatlösung extrahiert. Die organische Phase wird mit Natriumsulfat getrocknet und im Vakuum eingengt. Der Rückstand wird über Kieselgel chromatographiert (500 g Kieselgel 60, Laufmittel Essigester : Petrolether 6 : 4).

| | |
|---|---|
| Ausbeute | 1,4 g; 77,9% der Theorie. |
| DC | R_{f} = 0,15 (Essigester : Petrolether 6 : 4) |

¹H-NMR (CDCl₃) δ = 1,2 - 1,45 (3d, 9H); 2,48 (d, 2H); 2,6 (m, 2H); 3,2 - 3,6 (m, 3H); 3,7 (s, 3H); 3,8 - 4,6 (m, 4H); 5,92 (d, 1H); 7,0 - 7,4 (m, 5H) ppm.

### Herstellungsbeispiele:

### Beispiel 1:

### Methyl-(E)-7-{4-(4-fluorphenyl)-6-[1(S)-hydroxymethyl-ethyl]-2-isopropyl-5-methoxymethyl-pyrid-3-yl}-3(R),5(S)-dihydroxy-hept-6-enoat

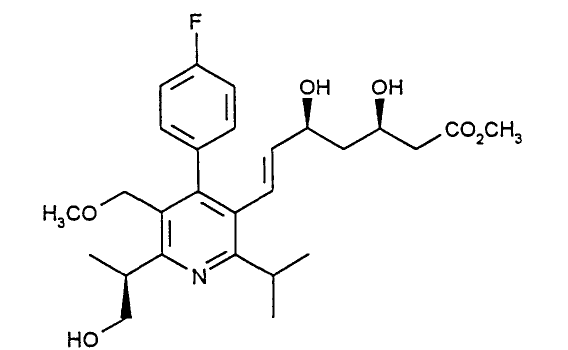

600 ml absolutes THF, 240 ml absolutes Methanol und 243,2 ml (0,24 mol) einer 1 molaren Triethylboranlösung in THF werden 1 Stunde bei Raumtemperatur gerührt. Nach Abkühlen auf -75°C (Innentemperatur, Aceton/Trockeneiskühlung) werden 59,2 g (0,12 mol) der Verbindung aus Beispiel XV gelöst in 150 ml absolutem THF zugegeben. Nach 30 min bei -75°C werden portionsweise 6,9 g (0,18 mol) Natriumborhydrid zugegeben und dann nochmals 3 Stunden bei -75°C gerührt. Man entfernt das Kältebad und tropft bei 0°C 100 ml gesättigte Ammoniumchloridlösung zu. Dann werden 700 ml Wasser und 500 ml Essigester zugegeben. Die wässrige Phase wird abgetrennt und zweimal mit je 200 ml Essigester gewaschen. Die vereinigten organischen Phasen werden mit 400 ml gesättigter Kochsalzlösung gewaschen, über Natriumsulfat getrocknet und dann im Vakuum eingeengt. Der Rückstand wird sechsmal in 500 ml Methanol gelöst und wieder am Rotationsverdampfer eingeengt und dann über Kieselgel chromatographiert (1,3 kg Kieselgel 60, Laufmittel Petrolether/Essigester 1:1). Die produkthaltigen Fraktionen werden eingeengt. Man erhält 50,5 g Rohprodukt, die nochmals über Kieselgel chromatographiert werden.

| | |
|---|---|
| Ausbeute: | 33,9 g; 57,8 % d. Th. |
| de = | 59 % (HPLC) |
| DC: | R_{f} = 0,14 (Petrolether/Essigester 1:1) |

¹H-NMR (CDCl₃): δ = 1,25 (2d, 6H, CH₃); 1,40 (m, 2H, -CH₂-); 1,43 (d, 3H, CH₃); 2,41 (m, 2H, -CH₂-); 3,18 (s, 3H, OCH₃); 3,2 - 3,4 (m, 2H, CH und CH-CH₂); 3,71 (s, 3H, OCH₃); 3,85 (m, 2H, CH₂-OH); 4,0 - 4,2 (m, 3H, CH₂O und CHO); 4,32 (m, 1H, CHO); 5,28 (dd, 1 H, =CH); 6,31 (d, 1H, =CH); 7,0 - 7,2 (m, 4H, Ar) ppm.

### Trennung der Diastereomeren der Verbindung aus Beispiel 1 (de = 59 %) durch präparative HPLC

30 g der Verbindung aus Beispiel 1 werden in 160 ml Ethanol p.a. (Merck) gelöst und mit 640 ml n-Heptan (LiChrosolv, Merck) verdünnt. Mittels Autoinjektor werden 940 Injektionen à 0,8 ml (30 mg) alle 15 min auf die HPLC-Säule gegeben und mit Hilfe eines Fraktionssammlers über eine Peak/Time-Steuerung 13 Fraktionen gesammelt. Nach der Reinheitskontrolle dieser Fraktionen mittels HPLC werden die Fraktionen 1 - 6 (Peak 1, Diastereomeres 1A), 7 + 8 (Gemisch A + B) und 9 - 13 (Peak 2, Diastereomeres 1B) jeweils vereinigt. Das Lösungsmittel wird am Rotationsverdampfer im Vakuum abdestilliert. Die Mischfraktionen werden nochmals analog getrennt.

| | |
|---|---|
| Ausbeute | 16,9 g 1A (de = 99,2 %), 76 % d. Th. bezogen auf 1 4 g 1B (de = 77,8 %), 70 % d. Th. bezogen auf 1. |

### Parameter der präparativen HPLC

| | |
|---|---|
| Apparat | Hochdruckpumpen Modell 305 und 306 (Gilson) |
| | Fraktionssammler Modell 201 (Gilson) |
| | Autoinjektor Modell 231 XL (Gilson) |
| | Detektor Modell SP 100 (Spectra Physics) |
| | Schreiber Modell 320 D (Servogor) |
| Säule | Länge: 250 mm; innerer Durchmesser: 20 mm; Temperatur: 40°C |
| Stationäre | |
| Phase | Chiralpak AS, No. 068-702-40914 (Daicel Chemical Ind.) |
| Eluent | n-Heptan (LiChrosolv, Merck) 95 %, Ethanol (p.a., Merck) 5 % |
| Fluß | 10 ml/min |
| Detektion | UV, 230 nm |
| Druck: | 2 x 10⁶ Pa |

### Beispiel 2:

### Natrium-(E)-7-{4-(4-fluorphenyl)-6-[1(S)-hydroxymethyl-ethyl]-2-isopropyl-5-methoxymethyl-pyrid-3-yl}-3(R),5(S)-dihydroxy-hept-6-enoat

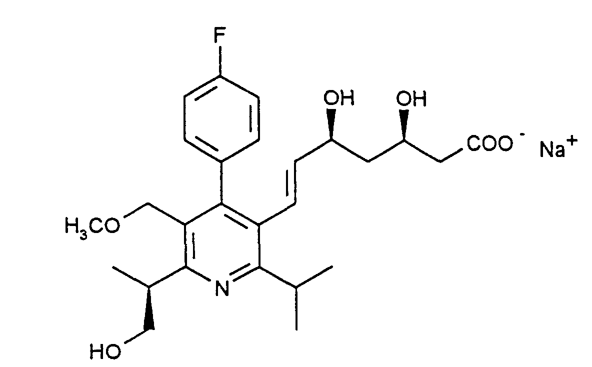

10,6 g (21,68 mmol) der Verbindung 1A werden in 150 ml THF gelöst. Bei Raumtemperatur werden 238,5 ml 0,1 molare Natronlauge zugegeben. Nach 1 Stunde bei Raumtemperatur wird das THF am Rotationsverdampfer abrotiert und der wässrige Rückstand gefriergetrocknet.

| | |
|---|---|
| Ausbeute | 10,7 g; 99,3 % d. Th. |

### Beispiel 3:

### Methyl-(E)-7-{6-[1(S)-hydroxy-methyl-ethyl]-5-hydroxymethyl-4-(4-fluorphenyl)-2-isopropyl-pyridin-3-yl}-(3R),5(S)-dihydroxy-hept-6-enoat

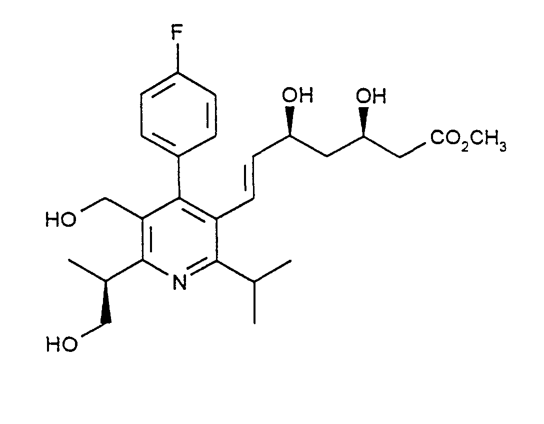

24 ml absolutes THF, 6 ml absolutes Methanol und 5,9 ml (5,92 mmol) einer 1 molaren Triethylboranlösung in THF werden 1 Stunde bei Raumtemperatur gerührt. Nach Abkühlen auf -75° C werden 1,4 g (2,96 mmol) der Verbindung aus Beispiel XX gelöst in 20 ml absolutem THF zugegeben. Nach 30 min bei -75° C werden portionsweise 168 mg (4,44 mmol) Natriumborhydrid zugegeben und dann wird nochmals 3 Stunden bei -75° C gerührt. Man entfernt das Kältebad und tropft bei 0° C 100 ml gesättigte Ammoniumchlorid-Lösung zu. Dann werden 100 ml Wasser und 100 ml Essigester zugegeben. Die wässrige Phase wird abgetrennt und zweimal mit je 100 ml Essigester gewaschen, mit Natriumsulfat getrocknet und dann im Vakuum eingeengt. Der Rückstand wird viermal in 100 ml Methanol gelöst und wieder am Rotationsverdampfer eingeengt und dann über Kieselgel chromatographiert (500 g Kieselgel 60, Laufmittel Essigester : Petrolether 6 : 4).

| | |
|---|---|
| Ausbeute | 1,16 g; 82,5% der Theorie. (de = 59 %, HPLC) |
| DC | R_{f} = 0,33 (Essigester/Petrolether 7:3) |

¹H-NMR (CDCl₃) δ = 1,15 - 1,3 und 1,4 (3d, 9H); 2,42 (m, 2H); 3,1 (m, 1H); 3,2 - 3,65 (m, 3H); 3,71 (s, 3H); 3,8 - 4,55 (m, 6H); 5,78 (dd, 1H); 6,3 (d, 1H); 7,0 - 7,25 (m, 4H) ppm.

Das reine Diastereomer wird wie in Beispiel 1 beschrieben durch präparative HPLC erhalten.

### Beispiel 4:

### Natrium-(E)-7-{6-[1(S)-hydroxy-methyl-ethyl]-5-hydroxymethyl-4-(4-fluorphenyl)-2-isopropyl-pyridin-3-yl}-(3R),5(S)-dihydroxy-hept-6-enoat

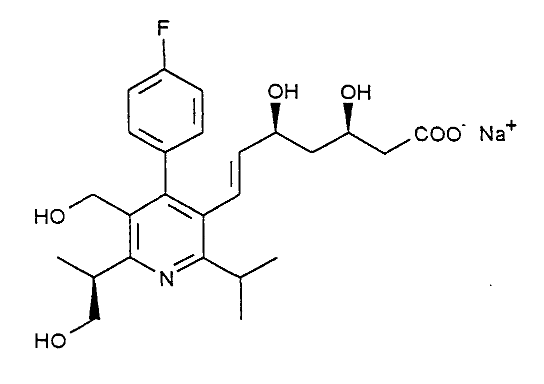

504 mg (1,06 mmol) der Verbindung aus Beispiel 3 werden in 10 ml THF gelöst. Bei Raumtemperatur werden 10,6 ml 0,1 molare Natronlauge zugegeben. Nach 1 Stunde bei Raumtemperatur wird das THF am Rotationsverdampfer abgezogen und der wässrige Rückstand gefriergetrocknet.
- Ausbeute:: 511 mg; 99,7% der Theorie. de > 99 % (HPLC)
¹H-NMR (CD₃OD): δ = 1,23, 1,25, 1,36 (3d, 9H); 1,26 (ddd, 1H); 1,51 (ddd, 1H); 2,17 (dd, 1H); 2,26 (dd, 1H); 3,45 (sept. 1H); 3,50 (m, 1H); 3,75 (m, 1H); 3,87 (m, 2H); 4,18 (m, 1H); 4,33 (m, 2H); 5,34 (dd, 1H); 6,29 (dd, 1H); 7,11 - 7,22 (m, 4H) ppm.

## Patentansprüche

1. 6-(Hydroxymethyl-ethyl)pyridine der allgemeinen Formel (I) 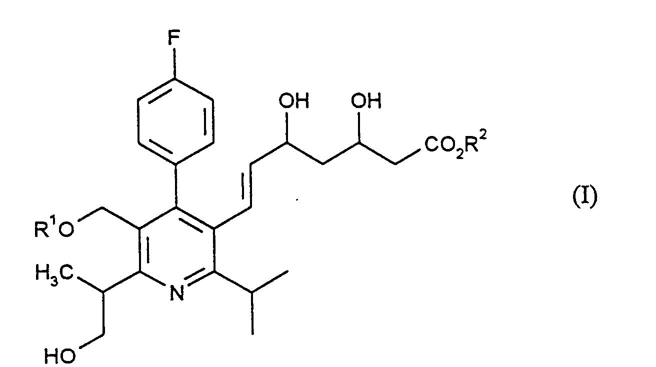 in welcher
R¹ für Wasserstoff oder für Methyl steht,
und
R² für Wasserstoff oder für Methyl steht,
und deren Salze.

2. 6-(Hydroxymethyl-ethyl)pyridine der Formel nach Anspruch 1 in der Erythro-Konfiguration.

3. 6-(Hydroxymethyl-ethyl)pyridine der Formel nach Anspruch 1 in Form der 1S- und 1R-Enantiomeren der (3R, 5S)-Dihydroxyheptensäure und Derivate in der Erythro-(E)-Konfiguration und deren Salze.

4. 6-(Hydroxymethyl-ethyl)pyridine nach Anspruch 1 bis 3 als Arzneimittel.

5. Verfahren zur Herstellung von 6-(Hydroxymethyl-ethyl)pyridinen nach Anspruch 1 bis 3, **dadurch gekennzeichnet, daß** man
Verbindungen der allgemeinen Formel (II) 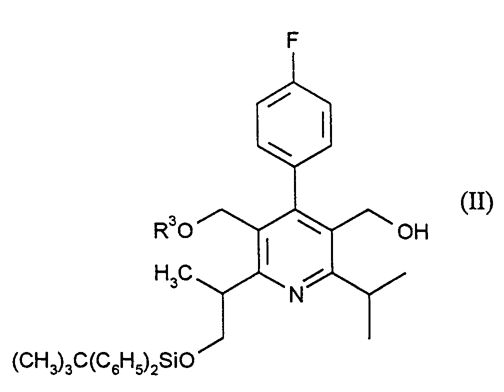 in welcher
R³ für Methyl oder für den Rest -Si(CH₃)₂C(CH₃)₃ (TBDMS) steht,
zunächst mit Aluminiumoxid und Pyridiniumchlorochromat in inerten Lösemitteln zu den Aldehyden der allgemeinen Formel (III) 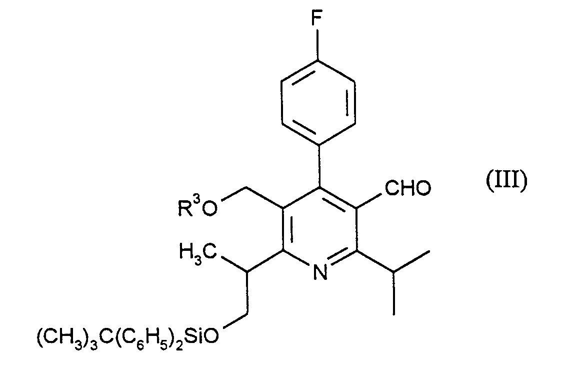 in welcher
R³ die oben angegebene Bedeutung hat,
oxidiert,
aus diesen in einem zweiten Schritt durch Umsetzung mit dem Ketophosphonat (CH₃O)₂PO-CH₂-CO-CH₂-CH(OSi(CH₃)₂)C(CH₃)₃)-CH₂-CO₂CH₃ in Anwesenheit von Basen und Lösemitteln
die Verbindungen der allgemeinen Formel (IV) 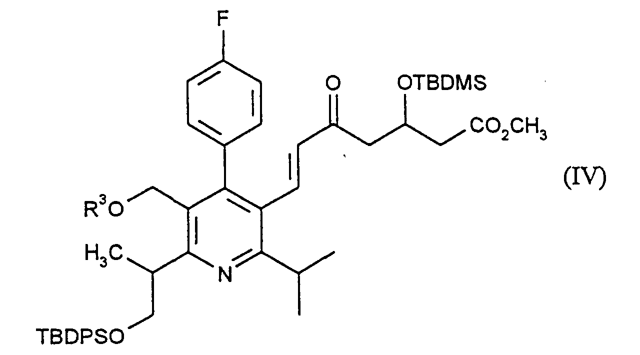 in welcher
TBDPS = (CH₃)₃C(C₆H₅)₂Si bedeutet,
und
R³ die angegebene Bedeutung hat,
herstellt,
diese anschließend durch Abspaltung der Hydroxyschutzgruppen TBPS in die Verbindungen der allgemeinen Formel (V) 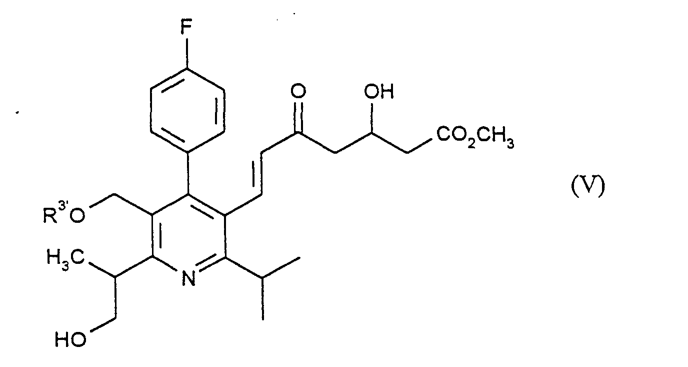 in welcher
R^{3'} die angegebene Bedeutung hat
überführt
und in einem letzten Schritt in inerten Lösemitteln mit Natriumborhydrid / Triethylboran die Ketogruppe reduziert,
und im Fall der Säuren die Ester hydrolysiert,
und gegebenenfalls Gemische von Diastereomeren durch Chromatographie oder Kristallisation trennt und in die enantiomerenreinen Verbindungen überführt.

6. Arzneimittel enthaltend mindestens ein 6-(Hydroxymethyl-ethyl)pyridin nach Anspruch 1 bis 3.

7. Arzneimittel nach Anspruch 6 zur Behandlung von Hyperlipoproteinämie.

8. Arzneimittel nach Anspruch 6 und 7 zur Behandlung von Arteriosklerose.

9. Verwendung von 6-(Hydroxymethyl-ethyl)pyridinen nach Anspruch 1 bis 3 zur Herstellung von Arzneimitteln.

10. Verwendung nach Anspruch 9 zur Herstellung von anti-arteriosklerotischen Arzneimitteln.

## Claims

1. 6-(Hydroxymethyl-ethyl)pyridines of the general formula (I) 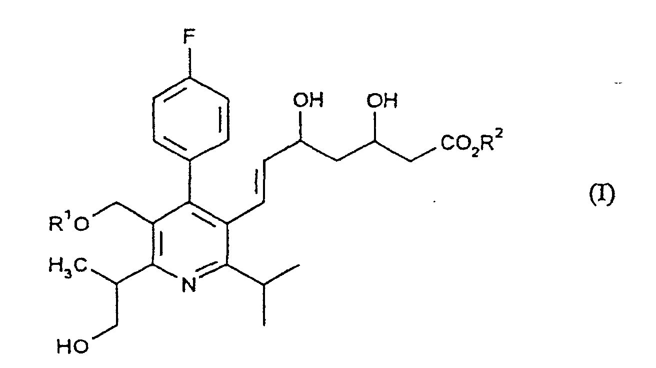 in which
R¹ represents hydrogen or methyl,
and
R² represents hydrogen or methyl,
and their salts.

2. 6-(Hydroxymethyl-ethyl)pyridines of the formula according to Claim 1 in the erythro configuration.

3. 6-(Hydroxymethyl-ethyl)pyridines of the formula according to Claim 1 in the form of the 1S and 1R enantiomers of (3R, 5S)-dihydroxyheptenoic acid and derivatives in the erythro(E) configuration and their salts.

4. 6-(Hydroxymethyl-ethyl)pyridines according to Claims 1 to 3 as medicaments.

5. Process for the preparation of 6-(hydroxymethyl-ethyl)pyridines according to Claims 1 to 3, **characterized in that**
compounds of the general formula (II) 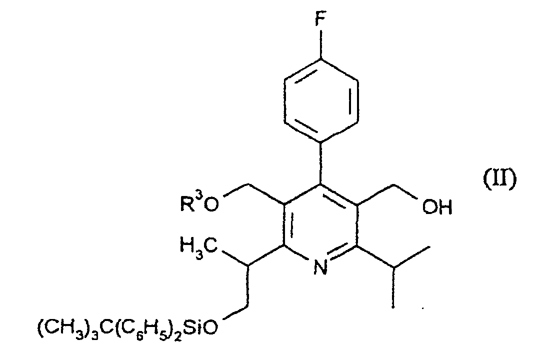 in which
R³ represents methyl or the radical -Si(CH₃)₂C(CH₃)₃ (TBDMS),
are first oxidized with aluminium oxide and pyridinium chlorochromate in inert solvents to give the aldehydes of the general formula (III) 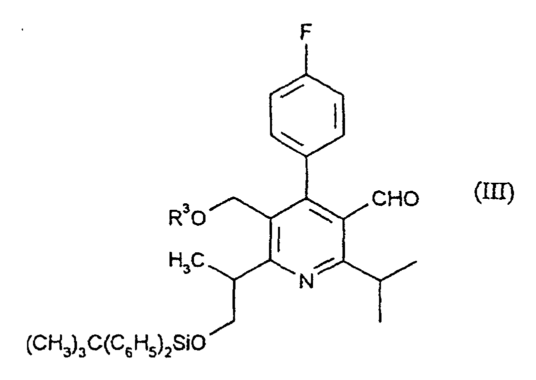 in which
R³ has the meaning indicated above,
from these in a second step by reaction with the ketophosphonate (CH₃O)₂PO-CH₂-CO-CH₂-CH(OSi(CH₃)₂)C(CH₃)₃)-CH₂-CO₂CH₃ in the presence of bases and solvents
the compounds of the general formula (IV) 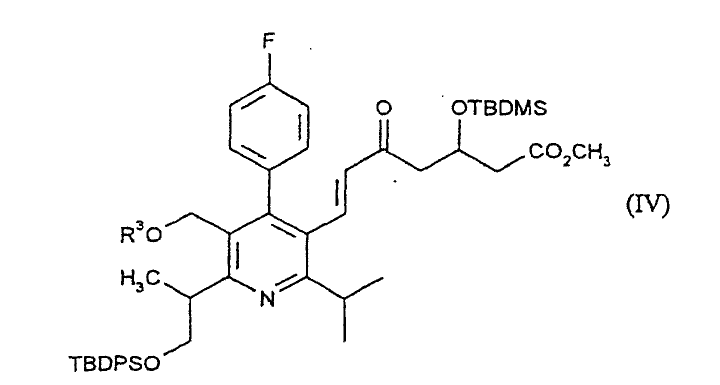 in which
TBDPS = (CH₃)₃C(C₆H₅)₂Si,
and
R³ has the meaning indicated,
are prepared,
these are then converted by removal of the hydroxyl protective groups TBPS into the compounds of the general formula (V) 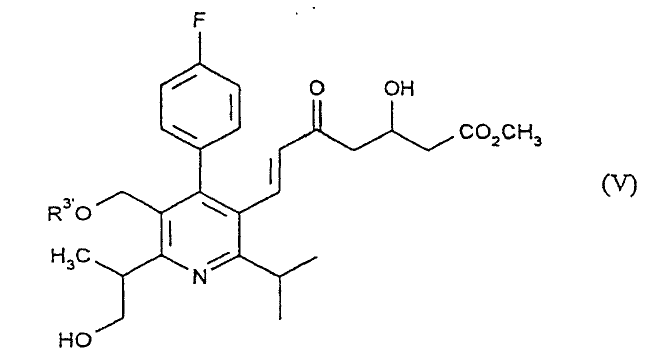 in which
R^{3'} has the meaning indicated
and in a last step the keto group is reduced in inert solvents using sodium borohydride/triethylborane,
and in the case of the acids the esters are hydrolysed,
and, if appropriate, mixtures of diastereomers are separated by chromatography or crystallization and converted into the enantiomerically pure compounds.

6. Medicaments comprising at least one 6-(hydroxymethyl-ethyl)pyridine according to Claims 1 to 3.

7. Medicaments according to Claim 6 for the treatment of hyperlipoproteinaemia.

8. Medicaments according to Claims 6 and 7 for the treatment of arteriosclerosis.

9. Use of 6-(hydroxymethyl-ethyl)pyridines according to Claims 1 to 3 for the production of medicaments.

10. Use according to Claim 9 for the production of antiarteriosclerotic medicaments.

## Revendications

1. 6-(hydroxyméthyléthyl)pyridine de formule générale (I) 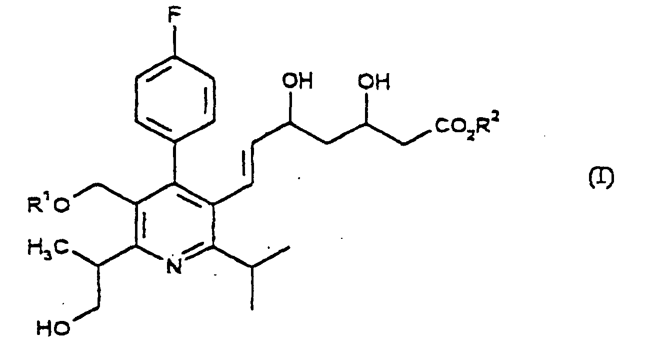 dans laquelle
R¹ désigne l'hydrogène ou un radical méthyle,
et
R² désigne l'hydrogène ou un radical méthyle,
et leurs sels.

2. 6-(hydroxyméthyl-éthyl)pyridine de formule I selon la revendication 1 en configuration érythro.

3. 6-(hydroxyméthyl-éthyl)pyridine de formule I selon la revendication 1 sous la forme d'énantiomères 1S et 1 R de l'acide (3R, 5S)-dihydroxyhepténoïque et ses dérivés en configuration érythro (E) et leurs sels.

4. 6-(hydroxyméthyl-éthyl)pyridine selon l'une des revendications 1 à 3 comme médicament.

5. Procédé de préparation de 6-(hydroxyméthyl-éthyl)pyridines selon l'une des revendications 1 à 3, **caractérisé en ce que**
des composés de formule générale (II) 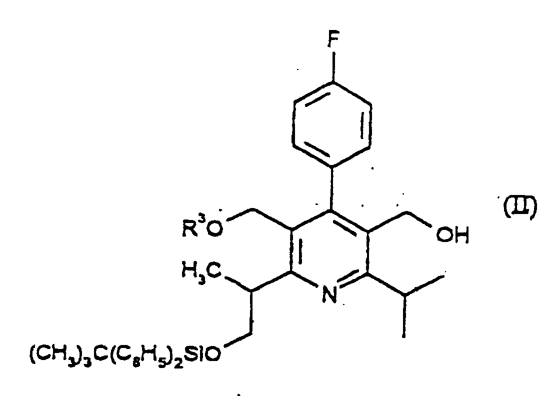 dans laquelle
R³ désigne un radical méthyle ou -Si(CH₃)₂C(CH₃)₃ (TBDMS),
sont d'abord oxydés avec de l'oxyde d'aluminium et du chlorochromate de pyridinium dans des solvants inertes en aldéhydes de formule générale (III) 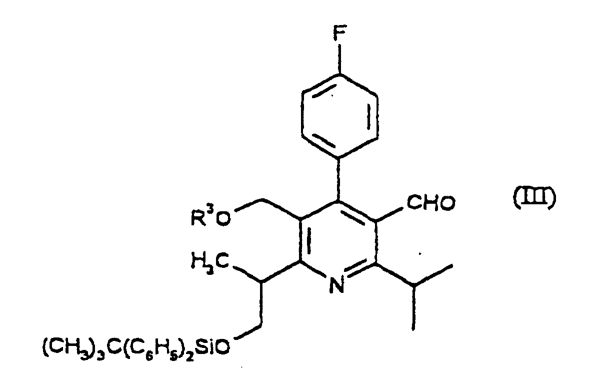 dans laquelle R³ a la signification indiquée précédemment,
à partir de ceux-ci, dans une deuxième étape par mise en réaction avec le cétophosphonate (CH₃O)₂PO-CH₂-CO-CH₂-CH(OSi(CH₃)₂)C(CH₃)₃)-CH₂-CO₂-CH₃ en présence de bases et de solvants
les composés de formule générale (IV) 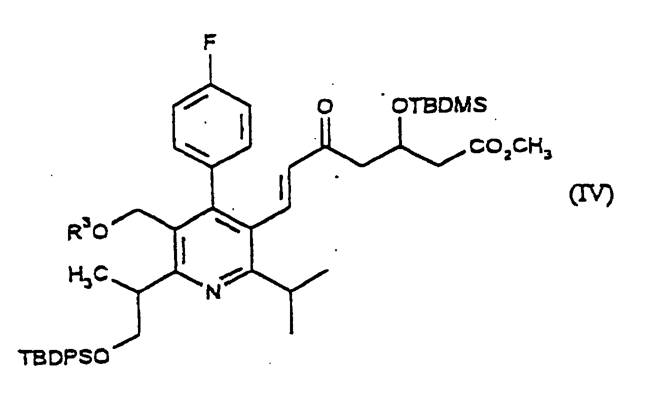 dans laquelle
TBDPS = (CH₃)₃C(C₆H₅)₂Si,
et
R³ a la signification indiquée,
sont préparés
ceux-ci sont ensuite transformés par séparation des radicaux TBPS protecteurs d'hydroxy en composés de formule générale (V) 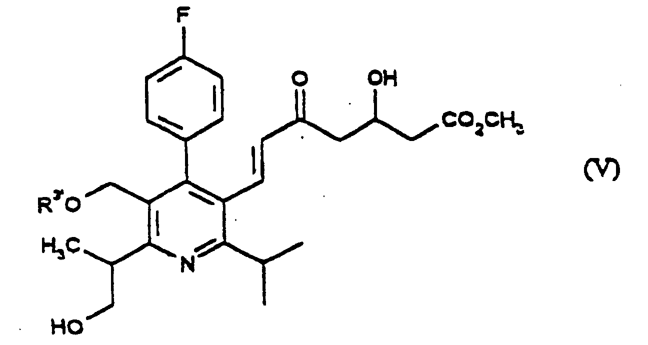 dans laquelle
R^{3'} a la signification indiquée
et, dans une dernière étape, le radical céto est réduit dans des solvants inertes avec de l'hydrure de bore-sodium / du triéthylborane et
dans le cas des acides, les esters sont hydrolysés,
et des mélanges de diastéréomères sont éventuellement séparés par chromatographie ou cristallisation et transformés en composés énantiomères purs.

6. Médicaments contenant au moins une 6-(hydroxyméthyléthyl)pyridine selon l'une des revendications 1 à 3.

7. Médicaments selon la revendication 6 de traitement d'une hyperlipoprotéinémie.

8. Médicaments selon l'une des revendications 6 et 7 pour le traitement de l'artériosclérose.

9. Mise en oeuvre de 6-(hydroxyméthyl-éthyl)pyridines selon l'une des revendications 1 à 3 pour la préparation de médicaments.

10. Mise en oeuvre selon la revendication 9 pour la préparation de médicaments anti-artérioscléreux.
